# EUROPEAN PATENT APPLICATION

(11) **EP 2 717 038 A1**
(43) Date of publication of application: **09.04.2014**
(21) Application number: 12793729.0
(22) Date of filing: 29.05.2012
(51) Int. Cl.: G01N 21/65

(54) **METHOD AND DEVICE FOR OPTICAL ANALYSIS OF BIOPOLYMER**

(30) Priority: 03.06.2011 JP 2011124871
(71) Applicant: Hitachi High-Technologies Corporation, Tokyo 105-8717 (JP)
(72) Inventor: TAKAHASHI Satoshi, Minato-ku, Tokyo 105-8717 (JP); KUMAZAKI Nobutaka, Minato-ku, Tokyo 105-8717 (JP)
(74) Representative: Moore, Graeme Patrick
(86) International application number: PCT/JP2012/063697
(87) International publication number: WO 2012/165400

(57) **Abstract**

The present invention relates to a biopolymer optical analysis apparatus provided with a biopolymer characteristic analysis chip 1100 including a solid substrate 1110, a plurality of nanopores 1120, 1121 provided in the solid substrate 1110, and electrically conductive thin films 1130a, 1130b, 1131a, and 1131b disposed on the solid substrate 1110, at least portions of the electrically conductive thin films 1130a, 1130b, 1131a, and 1131b facing the nanopores 1120, 1121, a light source and an irradiation optical system for producing Raman scattered lights from biopolymers entering the nanopores 1120, 1121, and a detection device including a Raman scattered light collecting system, a separating component that splits collected light into transmitted light and reflected light at different ratios in a particular wavelength range, an image-forming optical system through which the split lights form images on a detector, and a two-dimensional detector for detecting the lights forming the images. External light from the light source and the irradiation optical system is applied to the characteristic analysis chip 1100, and Raman scattered lights from the biopolymers in the analysis chip 1100 are detected in the detection device. Characteristics of monomer units constituting the biopolymers are analyzed from the results of detection.

## Description

### Technical Field

The present invention relates to an apparatus and a method for analyzing characteristics of a biopolymer by using a device in thin film form having nanosize pores. More particularly, the present invention relates to the structure of a device, an apparatus and a method for performing sequence analysis by forming a near field on a thin film and optically detecting a nucleic acid such as DNA or RNA without labeling the nucleic acid.

### Background Art

A technique using nanopores is attracting attention as an approach to realize a next-next-generation DNA sequencer. It is thought that the significant advantage of the nanopore method resides in enabling analysis of DNA without labeling the DNA, in other words, without using a reagent such as an enzyme or a fluorescent pigment. Nanopores are roughly divided into two kinds associated with their forming methods: a bionanopore provided by embedding a channel protein forming a nanosize opening (hereinafter referred to as "nanopore") in a bilayer membrane; and a solid-state nanopore formed by performing microfabrication on a semiconductor material.

As a DNA analysis method using such a nanopore, two kinds of methods have heretofore been proposed. One of them is a blockade current method. That is, liquid baths are provided on opposite sides of a thin film in which a nanopore is formed, and an electrolytic solution and electrodes are provided in each liquid chamber. When a voltage is applied between the electrodes, an ion current flows via the nanopore. The magnitude of the current as a first-order approximation is proportional to the sectional area of the nanopore. When DNA passes through the nanopore, the DNA closes the nanopore, the effective sectional area is reduced thereby and, as a result, the current is reduced. The amount of this reduction is called blockade current. A single-stranded DNA and a double-stranded DNA can be recognized as different from each other based on the magnitude of the blockade current. It has been reported that with a biopore in one form kinds of DNA bases can be identified from the magnitude of blockade current (Non Patent Literature 1, also referred to as "first related art" below). A bilayer membrane used for a biopore, however, is a delicate thin film formed by aggregation of organic low molecules by a weak force, and thought to have a mechanical stability problem. Also, the process of incorporating channel proteins in a bilayer membrane depends on a natural phenomenon and, therefore, entails a problem in terms of control of the number of channels and a problem in terms of reproducibility. On the other hand, solid-state nanopores use a semiconductor substrate or the like as a thin film and are, therefore, thought to be more advantageous in terms of structural stability than biopores. Also, since solid-state nanopores are mechanically formed, they are advantageous in being capable of being industrially produced. An apparatus and a method in which graphene is used as a thin film material other than the semiconductor substrate and in which a biomolecule passing through graphene in which a solid-state nanopore is provided is analyzed have also been reported (Patent Literature 1). However, no success in identifying kinds of DNA bases from the blockade current with a solid-state nanopore has been reported.

The second method is a tunnel current method. That is, a method has been proposed in which a pair of electrodes are opposedly disposed on a nanopore; a tunnel current between DNA passing through the nanopore and the electrodes is measured by applying a voltage between the electrodes; and the DNA is analyzed based on the magnitude of the tunnel current. With respect to a related art, it has been reported that when a tunnel current is measured by using a scanning probe microscope, dissolving in an organic solvent a nucleoside modified with a saccharide and introducing the nucleoside into the gap between nanogap electrodes, the average of the tunnel current varies depending on kinds of bases (Non Patent Literature 2, also referred to as "second related art" below). This art, however, has limitations in terms of experimental conditions in that the specimen is a nucleoside (having no phosphoric acid) and is not a chain nucleic acid; there is a need for modifying the specimen; there is a need for dissolving the specimen in an organic solvent; and a nanopore is not used, and has other problems in that there are tunnel current distributions having overlaps between kinds of bases and, therefore, the ability to identify the bases is low.

On the other hand, as another approach to measure a monomolecular nucleic acid without labeling the nucleic acid, a tip enhanced Raman scattering (TERS) method has been reported (Non Patent Literature 3, also referred to as "third related art" below). In this method, silver is formed on the tip of a probe of an atomic force microscope (AFM); a chain nucleic acid molecule fixed on a mica substrate is scanned with the AFM to obtain an AFM image of the nucleic acid; the probe is thereafter brought close to the nucleic acid; and laser light is applied. Then a local light enhancement field (near field) is formed at the probe tip to excite the nucleic acid. Raman scattered light radiated from the excited nucleic acid is spectroscopically measured to obtain a Raman scattering spectrum from the nucleic acid. Since the S/N of the obtained signal is higher than the number of nucleic acid bases contained in the near field, sensitivity capable of measuring one base can be obtained. Also, the Raman scattering spectrum has two-dimensional information, i.e., an intensity pattern with respect to the wave number, and therefore has the advantages of having an amount of information markedly larger than that of one-dimensional information such as a blockade current or a tunnel current and having a high qualitative discriminability. The size of the near field is determined by the radius of curvature of the probe tip, and the spatial resolution in the third related art is on the order of 10 nm. This corresponds to the length of about 30 bases in terms of the number of nucleic acid bases. The result obtained in this method contains overlapped information for a lot of bases. As a method for identifying information on each base from such overlapping information, a method has been proposed in which a nucleic acid is scanned with a probe along the chain of the nucleic acid for example, and inference of a base having entered the near field and a base having exited the near field from changes (differences) in the Raman scattering spectrum is repeated to obtain sequence information (hereinafter referred to as "differential method"). Fixing a nucleic acid on a solid substrate in advance is required for carrying out this method. Also, an AFM apparatus including a high-resolution slightly-movable stage is required for measurement and there is a need for a delicate operation to perform three-dimensional scanning with an AFM probe at a sub-nm accuracy. That is, there is a problem that the construction and the operation of the apparatus are complicated.

In a case where a plurality of DNA base sequences for example are simultaneously analyzed by using a device having a plurality of nanopores (multi-nanopore), there is a need to simultaneously detect signals generated from the plurality of nanopores. For example, a method has been reported in which target DNA is converted into fluorescent labeled oligonucleotides by using combinations of two color fluorescent lights corresponding to four bases; the fluorescent marked oligonucleotides are analyzed in nanopore arrays; and the base sequences of the target DNA are determined based on generated two-color fluorescent signals (Non Patent Literature 4). This method requires the operation to convert target DNA to the oligonucleotides for analysis and requires an increased cost because of use of fluorescent light.

### Citation List

### Patent Literature

Patent Literature 1: International Publication No. WO 2009/035647

### Non Patent Literature

Non Patent Literature 1: Clarke, J. et al., Nat. Nanotech., Vol. 4, pp. 265-270, 2009
Non Patent Literature 2: Chang, S. et al., Nano Lett., Vol. 10, pp. 1070-1075, 2010
Non Patent Literature 3: Bailo, E. et al., Angew. Chem. Int. Ed., Vol. 47, pp. 1658-1661, 2008
Non Patent Literature 4: McNally, B. et al., Nano Lett., Vol. 10, pp. 2237-2244, 2010

### Summary of Invention

### Technical Problem

The method of analyzing a biopolymer based on a blockaded current by using a biopore (the first related art) is of low mechanical stability and has difficulty in obtaining a reproducible result with stability. The method of analyzing a biopolymer based on a tunnel current by using a nanopore (the second related art) is of low base discriminability and has difficulty in reading the base sequence of a nucleic acid with high accuracy. The method of analyzing a biopolymer by using TERS (the third related art) requires a complicated apparatus construction and a delicate operation. Also, the related arts have difficulty in simultaneously measuring a plurality of specimens, for example, in making measurements on a plurality of nanopores.

Therefore, an object of the present invention is to provide an apparatus and a method for analyzing characteristics of a biopolymer with a simple arrangement having improved mechanical stability and high spatial resolution and sensitivity. Another object of the present invention is to provide an apparatus and a method for detecting signals generated from a plurality of nanopores simultaneously, i.e., at a high throughput, and with high accuracy.

### Solution to Problem

The inventors of the present invention eagerly made studies to solve the above-described problems and found, as a result of the studies, that the provision of suitable electrically conductive thin film suitably disposed on a substrate with a solid-state nanopore or the provision of a channel on a solid substrate enables increasing the spatial resolution and improving the sensitivity when characteristics of a biopolymer entering the nanopore or the channel are analyzed on the basis of Raman scattering, and also found that it is possible to detect signals generated from a plurality of nanopores at a high throughout by splitting the Raman scattered light at different ratios for each wavelength at the time of detecting the Raman scattered light, by detecting the split lights with a detector having a plurality of detection pixels, and by calculating the ratios of intensities at corresponding split image positions, and it is possible to analyze characteristics of biopolymers from the calculated ratios. The present invention has been completed on the basis of these findings. To be specific, the present invention includes the following.
[1] A biopolymer optical analysis apparatus provided with:
   a biopolymer characteristic analysis chip comprising a solid substrate, a plurality of nanopores provided in the solid substrate, and an electrically conductive thin film disposed on the solid substrate, wherein at least a portion of the electrically conductive thin film faces the nanopores;
   a light source and an irradiation optical system for producing Raman scattered lights from a biopolymer entering the nanopores; and
   a detection device comprising a Raman scattered light collecting system, a separating component that splits collected light into transmitted light and reflected light at different ratios in a particular wavelength range, an image-forming optical system through which the split lights form images on a detector, and a two-dimensional detector for detecting the lights forming the images,
   wherein external light is applied from the light source and the irradiation optical system to the characteristic analysis chip, and Raman scattered lights from the biopolymer in the analysis chip are detected in the detection device.
[2] A biopolymer optical analysis apparatus provided with:
   a biopolymer characteristic analysis chip comprising a solid substrate, a plurality of nanopores provided in the solid substrate, and an electrically conductive thin film disposed on the solid substrate, wherein at least a portion of the electrically conductive thin film faces the nanopores;
   a light source and an irradiation optical system for producing Raman scattered lights from a biopolymer entering the nanopores; and
   a detection device comprising a Raman scattered light collecting system, a separating component that splits collected light into transmitted light and reflected light at different ratios for each of prescribed wavelength bands, an image-forming optical system through which the split lights form images on a detector, and a two-dimensional detector for detecting the lights forming the images,
   wherein external light is applied from the light source and the irradiation optical system to the characteristic analysis chip, and Raman scattered lights from the biopolymer in the analysis chip are detected in the detection device.
[3] The biopolymer optical analysis apparatus according to [1] or [2], wherein the biopolymer is constituted by at least four kinds of monomers, and the separating component splits the light at different ratios such that at least four intensity ratios between the transmitted light and the reflected light are discriminated for each of the kinds of monomers constituting the biopolymer.
[4] The biopolymer optical analysis apparatus according to any one of [1] to [3], wherein the external light is applied to the electrically conductive thin film to cause the electrically conductive thin film to produce a near field at an end portion facing an opening portion of the nanopore, whereby Raman scattered light from the biopolymer entering the nanopore is produced.
[5] The biopolymer optical analysis apparatus according to any one of [1] to [4], wherein the electrically conductive thin film has an end portion having an acute angle, and the end portion having the acute angle is disposed so as to face the opening portion of the nanopore.
[6] The biopolymer optical analysis apparatus according to any one of [1] to [5], wherein the characteristic analysis chip has at least two electrically conductive thin films for each of the nanopores, and the at least two electrically conductive thin films are opposed to each other with the opening portion of the nanopore interposed therebetween.
[7] The biopolymer optical analysis apparatus according to any one of [1] to [6], wherein the electrically conductive thin film is formed of a metal.
[8] The biopolymer optical analysis apparatus according to any one of [1] to [6], wherein the electrically conductive thin film is formed of graphite.
[8-2] The biopolymer optical analysis apparatus according to [8], wherein the graphite is graphene.
[9] The biopolymer optical analysis apparatus according to any one of [1] to [8], wherein the thickness of the electrically conductive thin film is 0.1 nm to 10 nm.
[9-2] The biopolymer optical analysis apparatus according to any one of [1] to [9], wherein the solid substrate has thin film portions that substantially transmit light, and the nanopores are provided in the thin film portions.
[9-3] The biopolymer optical analysis apparatus according to any one of [1] to [9], wherein the electrically conductive thin film is disposed on a surface of the solid substrate.
[10] The biopolymer optical analysis apparatus according to any one of [1] to [9], wherein the electrically conductive thin film is disposed at an intermediate depth in a direction along a center axis of the nanopore in the solid substrate.
[11] The biopolymer optical analysis apparatus according to any one of [1] to [10], wherein a depth of each of the nanopores is equal to or larger than three times the monomer unit constituting the biopolymer.
[12] The biopolymer optical analysis apparatus according to any one of [1] to [11], further comprising a data processing section that analyzes characteristics of the biopolymer from the ratios of the intensities of Raman scattered light detected in the detection device.
[13] The biopolymer optical analysis apparatus according to any one of [1] to [12], further comprising a specimen moving mechanism that causes monomers in the biopolymer to enter the nanopore on a unit-by-unit basis.
[14] The biopolymer optical analysis apparatus according to any one of [1] to [13], wherein the biopolymer is selected from the group consisting of a nucleic acid, a peptide nucleic acid, a protein, a sugar chain, and an aptamer.
[15] The biopolymer optical analysis apparatus according to any one of [1] to [14], wherein analysis of characteristics of the biopolymer is determination of a base sequence of a nucleic acid.
[15-2] The biopolymer optical analysis apparatus according to any one of [1] to [15], further comprising a frame buffer memory in which measured values from the detection device are recorded.
[16] A biopolymer optical analysis apparatus provided with:
   a biopolymer characteristic analysis chip comprising a solid substrate and a plurality of pairs of electrically conductive thin films disposed on a surface of the solid substrate, wherein each pair of the electrically conductive thin films is disposed in a state of being opposed to each other with a certain distance;
   a light source and an irradiation optical system for producing Raman scattered lights from a biopolymer; and
   a detection device comprising a Raman scattered light collecting system, a separating component that splits collected light into transmitted light and reflected light at different ratios for each of prescribed wavelength bands, an image-forming optical system through which the split lights form images on a detector, and a two-dimensional detector for detecting the lights forming the images,
   wherein external light is applied from the light source and the irradiation optical system to the characteristic analysis chip, and Raman scattered lights from the biopolymer in the analysis chip are detected in the detection device.
[17] A biopolymer characteristic analysis method comprising the steps of:
   in the biopolymer optical analysis apparatus according to any one of [1] to [16], applying external light to a characteristic analysis chip to produce Raman scattered lights from a biopolymer entering nanopores;
   detecting the Raman scattered lights in a detection device; and
   analyzing characteristics of the biopolymer on the basis of results of detection of the Raman scattered lights.
[18] The biopolymer characteristic analysis method according to [17], wherein the biopolymer is selected from the group consisting of a nucleic acid, a peptide nucleic acid, a protein, a sugar chain, and an aptamer.
[19] The biopolymer characteristic analysis method according to [17] or [18], wherein a base sequence of a nucleic acid is determined.
[19-2] The biopolymer characteristic analysis method according to any one of [17] to [19], wherein the biopolymer exists in a specimen solution containing a second polymer that cannot enter the nanopore.
[19-3] The biopolymer characteristic analysis method according to any one of [17] to [19], wherein the kinds of monomers constituting the biopolymer are identified by comparing the intensity ratio between the transmitted light and the reflected light of the Raman scattered light with a reference or a control.

### Advantageous Effects of Invention

The present invention provides an apparatus and method for optical analysis of a biopolymer. The analysis apparatus of the present invention is based on a solid-state nanopore method and therefore has high structural stability and high reliability and is capable of two-dimensionally analyzing characteristics of a biopolymer with high spatial resolution and high sensitivity through Raman scattering. The analysis apparatus and the analysis method of the present invention are capable of analyzing a plurality of biopolymers in a plurality of nanopores two-dimensionally disposed simultaneously and are, therefore, simple and convenient and have a high throughput.

### Brief Description of Drawings

[Figure 1] Figure 1 is a schematic view of a nanopore chip for analysis of biopolymer characteristics.
[Figure 2] Figure 2 is a schematic enlarged view of a section of the nanopore chip.
[Figure 3] Figure 3 is a schematic construction diagram of a biopolymer optical analysis apparatus.
[Figure 4] Figure 4 is a sectional view schematically showing a construction of a specimen cell.
[Figure 5] Figure 5 shows a schematic diagram (A) showing the structure of a triangular metal thin film having an acute angle, and results of a simulation (B) of a near field produced in the vicinity of the metal thin film.
[Figure 6] Figure 6 shows a schematic diagram (A) showing the structure of two triangular metal thin films, and results (B) of a simulation of a near field produced in the vicinity of the metal thin films.
[Figure 7] Figure 7 shows typical Raman scattering spectra of nucleic acid bases.
[Figure 8] Figure 8 shows spectral distributions (A and B) of two split images (transmitted light and reflected light) detected in embodiment 6, and results (C) of ratio calculation for each kind of base.
[Figure 9] Figure 9 is a schematic view of a modified example of the nanopore chip for analysis of biopolymer characteristics.
[Figure 10] Figure 10 is a schematic enlarged view of a section of the nanopore chip in the modified example.
[Figure 11] Figure 11 is a schematic view of a multi-nanopore chip in embodiment 2.
[Figure 12] Figure 12 is a schematic construction diagram of a biopolymer optical analysis apparatus using the multi-nanopore chip.
[Figure 13] Figure 13 is a schematic enlarged view of a section of a nanopore chip in embodiment 3.
[Figure 14] Figure 14 is a schematic view of a nanopore chip in embodiment 4.
[Figure 15] Figure 15 is a schematic view of a nanopore chip in embodiment 5.
[Figure 16] Figure 16 is a schematic enlarged view of a section of the nanopore chip in embodiment 5.
[Figure 17] Figure 17 is a schematic construction diagram of a detection device in embodiment 6.
[Figure 18] Figure 18 is a characteristic diagram of a filter and mirror in embodiment 6.
[Figure 19] Figure 19 shows another example (schematic diagram) of a dichroic mirror in embodiment 6.
[Figure 20] Figure 20 is a schematic construction diagram of a detection device in embodiment 7.
[Figure 21] Figure 21 is a schematic construction diagram of a characteristic analysis chip including a channel having a projection structure.
[Figure 22] Figure 22 is a sectional view schematically showing the construction of a specimen cell.
[Figure 23] Figure 23 is a schematic construction diagram of a modified example of the characteristic analysis chip including a channel having a projection structure.
[Figure 24] Figure 24 is a schematic construction diagram of a characteristic analysis chip including a channel having one row of metal nanoparticles.
[Figure 25] Figure 25 is a schematic construction diagram of a characteristic analysis chip including a channel having one row of metal nanostructures.
[Figure 26] Figure 26 is a schematic construction diagram of a characteristic analysis chip including a channel having a plurality of rows of metal nanoparticles.
[Figure 27] Figure 27 is a schematic construction diagram of a characteristic analysis chip including a channel having a plurality of rows of metal nanostructures.

### Description of Embodiments

The present invention will be described in detail below. This application claims the benefit of priority from the prior Japanese Patent Application No. 2011-124871, filed on June 3, 2011, and includes the contents of the specification and/or drawings in the above patent application.

The present invention relates to a biopolymer optical analysis apparatus comprising: a device (a biopolymer characteristic analysis chip) for analyzing characteristics of a biopolymer by using a nanopore and Raman scattering (preferably, tip enhanced Raman scattering: TERS); a light source and an irradiation optical system for producing Raman scattered light; and a detection device for detecting Raman scattered light, and to a method of analyzing characteristics of a biopolymer by using the analysis apparatus. In the analysis apparatus of the present invention, external light is applied from the light source and the irradiation optical system to the characteristic analysis chip, and Raman scattered light from a biopolymer, produced on the analysis chip, is detected by the detection device.

The biopolymer characteristic analysis chip (hereinafter referred to as "analysis chip") will first be described. The analysis chip comprises a solid substrate, at least one nanopore provided in the solid substrate, and at least one electrically conductive thin film disposed on the solid substrate.

The biopolymer characteristic analysis chip may alternatively be a chip comprising a solid substrate and at least one channel provided in the solid substrate.

The solid substrate can be formed of an electrically insulating material, e.g., an inorganic material and an organic material (including a polymer material). Examples of the electrically insulating material are silicon, glass, quartz, polydimethylsiloxane (PDMS), polytetrafluoroethylene (PTFE), polystyrene and polypropylene. If a nanopore can be provided in the solid substrate, the size and thickness of the solid substrate are not particularly restricted. The size and thickness of the solid substrate are adjusted for fitting to components of the analysis apparatus (including a detector) used at the time of analysis of a biopolymer described later. The solid substrate can be made by a method well known in the present technical field. The solid substrate can alternatively be purchased as an article on the market. For example, the solid substrate can be made by using a technique such as photolithography, etching, laser abrasion, injection molding, casting, molecular beam epitaxy, chemical vapor deposition (CVD), or an electron beam or focused ion beam technique. The solid substrate may be coated in order to avoid adsorption of foreign molecules to its surface.

Preferably, the solid substrate has a thin film portion for provision of a nanopore or a channel. That is, the provision of a thin film portion on the solid substrate using a suitable material with a thickness suitable for forming a nanosize hole or channel enables a nanopore or a channel to be easily and efficiently formed on the solid substrate. Such a thin film portion may be formed of the same material as the solid substrate or may be formed of a different electrically insulating material. From the viewpoint of forming a nanopore or a channel, it is preferable that the material of the thin film portion be silicon oxide (SiO₂), silicon nitride (SiN), silicon oxynitride (SiON), a metal oxide or a metal silicate. Also, from the viewpoint of excitation efficiency and collection efficiency with respect to external light described later, it is preferable that the thin film portion (the entire solid substrate in some case) be substantially transparent. "Substantially transparent" means that about 50% or more, preferably 80% or more of external light can be transmitted. The thin film portion may be a single layer or a multilayer. In a case where the thin film portion is a multilayer, an electrically conductive thin film described later may be sandwiched between layers in the thin film portion. The thickness of the thin film portion of the solid substrate is 10 nm to 200 nm, preferably 15 nm to 100 nm, more preferably 20 nm to 50 nm. The thin film portion can be formed on the solid substrate by a technique well known in the present technical field, e.g., low-pressure chemical vapor deposition (LPCVD).

In the solid substrate, at least one nanopore, preferably two or more nanopores are provided. In the present invention, each of "nanopore" and "pore" is a hole of a nanometer (nm) size formed through the solid substrate, preferably through the thin film portion of the solid substrate between the front and back surfaces of the substrate. That is, the analysis chip used in the present invention is classified as a solid-state nanopore. In the present invention, "opening" denotes a portion of a pore exposed out of a solid substrate surface. At the time of analysis of characteristics of a biopolymer, a biopolymer or an ion in a specimen solution enters the nanopore through an opening on one side and exits the nanopore through an opening on the same side or on the opposite side.

A suitable size for the pore (i.e., opening size) according to the kind of a biopolymer to be analyzed can be selected. For example, the pore size is 1 nm to 100 nm, preferably 1 nm to 50 nm. More specifically, the pore size is, for example, equal to or larger than 1 nm and equal to or smaller than 2 nm, equal to or larger than 3 nm and equal to or smaller than 5 nm, or equal to or larger than 10 nm and equal to or smaller than 50 nm. The diameter of an ssDNA (single-stranded DNA) is about 1.5 nm. A suitable range of the pore diameter for analyzing an ssDNA is about 1.5 nm to 10 nm, preferably about 1.5 nm to 2.5 nm. The diameter of a dsDNA (double-stranded DNA) is about 2.6 nm. A suitable range of the pore diameter for analyzing a dsDNA is about 3 nm to 10 nm, preferably about 3 nm to 5 nm. Also in the case of analyzing a different biopolymer, e.g., a protein or a sugar chain, a pore diameter according to the outside diameter size of the biopolymer can be selected. The depth of the nanopore can be adjusted by adjusting the thickness of the solid substrate or the thin film portion of the solid substrate. The depth of the nanopore is at least two times, preferably, three times, more preferably five times larger than the monomer unit constituting a biopolymer. For example, in a case where a nucleic acid is selected as a biopolymer, it is preferable to set the depth of the nanopore equal to or larger than a value corresponding to three bases, e.g., about 1 nm or more. As a result, a biopolymer can be caused to enter the nanopore while the shape and the speed of movement of the nanopore are being controlled, thus enabling high-sensitivity and high-accuracy analysis. The shape of the pore is basically circular. However, the pore can alternatively be made elliptical or polygonal.

At least one pore can be provided in the solid substrate. In a case where a plurality of pores are provided, it is preferable to regularly arrange the pores. The intervals at which a plurality of pores are disposed can be set to 0.1 µm to 10 µm, preferably 0.5 µm to 4 µm according to the capacities of a detector, a light collecting system and a separating component to be used. The pore can be formed in the solid substrate by a method well known in the present technical field, for example, by applying an electron beam from a transmission electron microscope (TEM) and by using a nanolithography technique, an ion beam lithography technique, or the like.

At least one electrically conductive thin film is disposed on the solid substrate. The electrically conductive thin film can be formed of a material having electrical conductivity or a light-scattering characteristic. Examples of such a material are metals, e.g., platinum group metals, such as platinum, palladium, rhodium and ruthenium, gold, silver, copper, aluminum and nickel; and graphite, e.g., graphene (either a single layer or a multilayer may suffice).

The thickness of the electrically conductive thin film is set to 0.1 nm to 10 nm, preferably 0.1 nm to 7 nm according to the material to be used. If the thickness of the electrically conductive thin film is reduced, a near field to be generated can be restricted to enable analysis at a high resolution and high sensitivity. The size of the electrically conductive thin film is not particularly restricted. A suitable size for the electrically conductive thin film can be selected, for example, according to the sizes of the solid substrate and the nanopore to be used and the wavelength of excitation light to be used.

The electrically conductive thin film may be formed into any shape, provided that the thin film is capable of generating and enhancing a near field when irradiated with external light. A probe capable of generating such a near field is well known in the present technical field. For example, a shape having an acute-angle end portion and capable of generating and enhancing a near field by tip enhanced Raman scattering (TERS) and a metal bow-tie structure are known.

In a case where the electrically conductive thin film has a shape having an acute-angle end portion, the angle of the end portion is 10 to 80 degrees, preferably 20 to 60 degrees, more preferably 20 to 40 degrees. For a preferable shape of the electrically conductive thin film (light-scattering member) for forming such near-field light, a reference should be made, for example, to Japanese Patent Publication (Kokai) No. 2009-150899. The tip of the end portion of the electrically conductive thin film may be not a point in a strict sense. It may have, for example, a rounded shape having a radius of curvature equal to or smaller than a certain value, preferably equal to or smaller than 10 nm. The shape of the portion of the electrically conductive thin film other than the acute-angle end portion is not particularly restricted. For the other portion, any shape, e.g., a circular shape with no angled portion may be freely selected, provided that in the case of having angled portions, the angles other than the angle at the tip of the end portion are larger than this angle. Also, the entire electrically conductive thin film may be of any shape, provided that an acute-angle end portion is provided. It is possible to form the entire electrically conductive thin film into a polygonal shape such as a triangular, rectangular or pentagonal shape, a fan shape, or a shape formed by combining a circular shape and triangular shape.

On the other hand, it is also possible to adopt a metal bow-tie structure as a shape of the electrically conductive thin film. That is, two circular, elliptic or polygonal pieces of electrically conductive thin film are disposed so that projecting portions of their shapes are opposed to each other. For such a metal bow-tie structure, a reference should be made, for example, to U.S. Patent No. 6,649,894. The metal bow-tie structure can be regarded as a structure in which a gap (opening) is inserted in a region where a near field is formed. Anisotropy is introduced by the insertion of the gap to improve the detection sensitivity. For a description of such a technique, a reference should be made, for example, to U.S. Patent Nos. 6,768,556, and 6,949,732.

The electrically conductive thin film may be disposed on a surface of the solid substrate or may be disposed between solid substrates, provided that at least a portion of the thin film faces the nanopore. For example, the electrically conductive thin film may be disposed on a surface of the solid substrate so as to face the opening portion of the nanopore. The electrically conductive thin film may alternatively be disposed at a generally middle position (depth) in a direction along the center axis of the nanopore on the solid substrate. In such a case, it is preferable to provide a structure in which the electrically conductive thin film is sandwiched between thin film portions of the solid substrate. A near field is thereby formed at about the middle in the direction along the center axis of the nanopore (depth direction). As a result, Raman scattered light from a biopolymer can be produced in the nanopore while the shape and the speed of movement of the biopolymer are being controlled, thus enabling analysis with high accuracy and high sensitivity. When the electrically conductive thin film is disposed on the solid substrate, it is preferable to dispose the thin film by considering the direction of polarization of applied external light.

As long as at least one electrically conductive thin film is disposed for each nanopore, the number thereof may be odd or even. For example, it is possible to dispose one, two, three, four or more electrically conductive thin films for each nanopore. As described later with respect to an embodiment, it is preferable to dispose two or more electrically conductive thin films for each nanopore because if a plurality of electrically conductive thin films exist a strong field of light is formed. It is possible to alternatively provide one thin film having a plurality of units each corresponding to the above-described shape. As a concrete example of this, a thin film structure such as that in embodiment 5 in which two units are joined to each other may be provided.

It is preferable that the electrically conductive thin film be disposed in such a manner that its end portion faces the opening portion of the nanopore. More specifically, the electrically conductive thin film is disposed in a plane perpendicular to the nanopore center axis, with the end portion of the thin film facing the opening portion of the nanopore. In a case where at least two electrically conductive thin films are disposed, it is preferable that the electrically conductive thin films be disposed by being opposed to each other with the opening portion of the nanopore interposed therebetween. In such a case, the electrically conductive thin film is irradiated with external light to generate a near field at the end portion facing the nanopore, thereby producing Raman scattered light from a biopolymer entering the nanopore.

The electrically conductive thin film can be made and disposed on the solid substrate by a method well known in the present technical field. For example, in a case where the electrically conductive thin film is formed of silver, silver thin film of a desired thickness can be formed into a desired shape with an electron beam after being formed on the substrate by sputtering. In a case where the electrically conductive thin film is formed of a single layer of graphene, graphene made from graphite can be formed into a desired shape by being irradiated with an electron beam after being placed on a supporting substrate.

By irradiation of the above-described analysis chip with external light, a biopolymer entering the above-described nanopore is excited to produce Raman scattered light. Characteristics of the biopolymer can be analyzed based on results of detection of the Raman scattered light. Preferably, a near field is formed at the end portion of the electrically conductive thin film facing the opening portion of the nanopore by irradiating the electrically conductive thin film on the analysis chip with external light, and the biopolymer is two-dimensionally scanned with the near-field light. The thickness of the near field formed is basically the same as the thickness of the electrically conductive thin film. That is, since the electrically conductive thin film is perpendicular to the center axis of the nanopore, the thickness of the formed near field along the center axis direction is substantially equal to the thickness of the electrically conductive thin film. Therefore, a biopolymer can be analyzed at a high spatial resolution and high sensitivity by using the above-described analysis chip.

Detection of Raman scattered light from a biopolymer may be performed by introducing the biopolymer into a space, i.e., a channel, between the pair of electrically conductive thin films disposed, instead of the nanopore, and by moving the biopolymer along the solid surface. Preferably, the pair of electrically conductive thin films forming the channel is disposed by being spaced apart from each other by a distance such that a biopolymer can be introduced, and suitable for Raman scattering. Preferably, the channel is formed by the electrically conductive thin film. The channel, however, may be formed of a material other than the electrically conductive thin film. Also, a person skilled in the art can suitably design the channel, for example, with respect to the width and the shape by considering the kind and size of a biopolymer to be analyzed, the near field to be generated and other factors.

In one embodiment, a projecting shape having a pointed end is formed at least in one place in the channel. Any number of projecting shapes in any form may be formed at any positions if they are capable of generating a near field. For example, projecting shapes corresponding to the shapes of the electrically conductive thin film described above in relation to the nanopore may be formed. As one example, a projecting shape may be formed on one of two wall surfaces of the channel on opposite sides of the flow direction of the channel, or projecting shapes may be formed by being opposed from the two wall surfaces of the channel on opposite sides of the flow direction of the channel.

In another embodiment, one or more rows of metal nanoparticles or metal nanostructure may be fixed at least in one place in the channel across the channel. Any number of metal nanoparticles or metal nanostructures in any form formed of any material may be formed at any positions if they are capable of generating a near field.

By irradiation of the above-described analysis chip with external light, a biopolymer entering the above-described channel is excited to produce Raman scattered light. Characteristics of the biopolymer can be analyzed based on results of detection of the Raman scattered light. Preferably, a near field is produced at the projecting shape, metal nanoparticles or metal nanostructure portion in the channel in the analysis chip by irradiating this portion with external light, and the biopolymer is two-dimensionally scanned by causing Raman scattering with the near field. The thickness of the near field formed is basically the same as the thickness of the channel. Therefore, a biopolymer can be analyzed at a high spatial resolution, high sensitivity and a high S/N ratio by using the above-described analysis chip.

The light source and the irradiation optical system for causing Raman scattered light will next be described.

As the light source, a light source that applies external light (excitation light) of a wavelength such that Raman scattered light can be produced and that is well known in the present technical field may be used. For example, as the light source, a krypton (Kr) ion laser, a neodymium (Nd) laser, an argon (Ar) ion laser, a YAG laser, a nitrogen laser, a sapphire laser or the like may be used, though not exclusively. The light source applies external light having a wavelength of about 400 to 900 nm. The Raman scattered light produced is light obtained by ordinary Raman scattering, resonance Raman scattering, tip enhanced Raman scattering (TERS), surface enhanced Raman scattering (SERS), or the like.

It is preferable to use an irradiation optical system having a lens (a lens and an objective lens), a mirror (a half mirror, a dichroic mirror, or the like), a beam expander and an ND filter in combination with the light source in order to apply external light from the light source to the analysis chip and to converge the external light on the analysis chip. The irradiation optical system may be combined with other components including a filter (a band-pass interference filter or the like) or a filter unit, a confocal pinhole, a light shielding plate and an absorption end in order to reduce a background signal. Such an arrangement for producing Raman scattered light is well known in the present technical field, and a person skilled in the art can select preferable components as desired.

The detection device will subsequently be described. The detection device includes a filter that cuts excitation light, a Raman scattered light collecting system, a separating component that splits collected light into transmitted light and reflected light at different reflectances in a particular wavelength range, an image-forming optical system that forms images on a two-dimensional detector by the split light, and the two-dimensional detector for detecting the image-forming light.

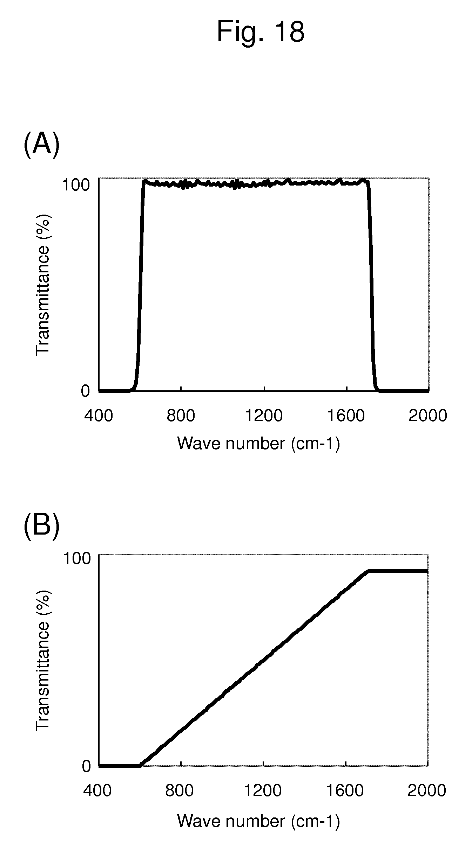

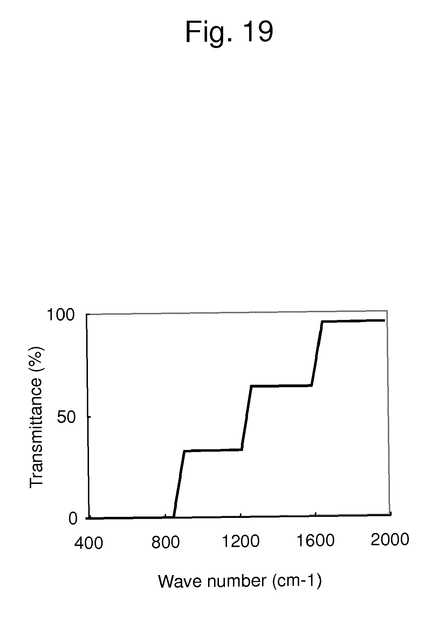

As the Raman scattered light collecting system, one or a plurality of objective lens and/or one or a plurality of mirrors may be used. One or a plurality of filters may be provided if necessary. The separating component is not particularly specified if it is capable of splitting light into transmitted light and reflected light at different ratios (reflectances) with respect to wavelengths. The separating component may split light at different ratios throughout the entire particular wavelength range. For example, a dichroic mirror or any other mirror having a similar characteristic may be used. Figure 18(B) shows an example of a characteristic of a dichroic mirror, which allows light to be transmitted (or reflected) at a higher ratio if the wave number of the light is higher. Figure 19 shows an example of a characteristic of another mirror, which allows light to be transmitted (or reflected) at ratios differing in steps with wave numbers corresponding to characteristic Raman scattering peaks. Preferably, the separator splits light at different ratios in a particular wavelength range such that the number of intensity ratios between transmitted light and reflected light corresponds to at least the number of kinds of monomers constituting a biopolymer (e.g., at least four kinds) so that the kinds of monomers can be distinguished from one from another.

The image-forming optical system is not particularly specified if it is capable of forming images with light on the two-dimensional detector to be used. For example, the image-forming optical system may use one or a plurality of image-forming lenses (including an achromatic lens).

The two-dimensional detector can output formed optical images as two-dimensional images by converting the optical images into electrical signals. One or a plurality of two-dimensional detectors may be used according to the number of nanopores and the disposition of the nanopores or the shape of the channel in the analysis chip to be used. Examples of such a two-dimensional detector are a charge-coupled device (CCD) area sensor, a complementary metal-oxide-semiconductor (CMOS) area sensor, and an area sensor using any other high-sensitivity element. The two-dimensional detector to be used may have a suitable pixel size and a suitable number of pixels. The type of the two-dimensional detector (e.g., a back-illuminated type or a front-illuminated type) is not particularly specified. Preferably, the two-dimensional detector has pixels for respectively detecting transmitted light and reflected light split at different ratios. Preferably, to prevent a reduction in sensitivity with speed-up of detection, the two-dimensional detector has a photomultiplication mechanism, e.g., an image intensifier, or uses an electron multiplier CCD camera (EM-CCD) having a signal multiplying function inside the device, or the like. The frame rate (transfer rate) of the two-dimensional detector is particularly preferably 1 kHz or higher.

Preferably, the detection device includes a large-capacity memory capable of directly recording Raman scattered light image information. The provision of such a memory enables high-speed analysis without intermediary devices such as a cable, a board and a computer. Preferably, for example, the analysis apparatus of the present invention further includes a frame buffer memory for recording measurement values from the detection device. The analysis apparatus may be connected to an output device (e.g., a computer, a monitor) that digitizes measurement values from the detection device and outputs the digitized measurement values.

In the detection device, as described above, Raman scattered light is collected by the light collecting system and split into transmitted light and reflected light, for example, at different reflectances with respect to wavelengths in the separating component, and images are formed by the split light and detected by the two-dimensional detector. The construction of such a detection device and a method of operating the detection device are described, for example, in International Publication No. WO 2010/150468 and US 2012/097864 A1. In embodiments in this specification, such a detection device is referred to as "two-split-ratio spectroscopic detection device".

In the present invention, Raman scattering is measured as two split two-dimensional images without undergoing wavelength dispersion and, therefore, Raman scattered light from a plurality of nanopores two-dimensionally disposed at a high density or from channels can be simultaneously detected, and high-throughput analysis of biopolymers passing through the plurality of nanopores or channels is enabled.

Preferably, the analysis apparatus of the present invention further includes a data processing section that analyzes characteristics of a biopolymer from the ratio of intensities of Raman scattered light detected in the detection device. Such a data processing section is, for example, a computer unit.

Preferably, the analysis apparatus of the present invention also includes a mechanism for controlling the speed of movement of a biopolymer, i.e., a specimen moving mechanism. The specimen moving mechanism causes monomers in a biopolymer to enter the nanopore or the channel in the analysis chip unit by unit, for example, by predetermined timing. As such a mechanism, a specimen drive device (an arbitrary function generator, electrodes) for driving a biopolymer by electrophoresis may be used. With such a specimen moving mechanism, the movement of a biopolymer can be controlled so that monomers in the biopolymer enter the nanopore or the channel and come off the same one after another, enabling detection of Raman scattered light corresponding to the each monomer (constituent unit) with respect to time.

As a method for controlling the speed of movement of a biopolymer, a method of increasing the viscosity of a specimen solution containing a biopolymer is conceivable. For example, by controlling the temperature around the analysis chip, the temperature of a specimen solution is reduced and the viscosity of the specimen solution is increased, thereby limiting the Brownian movement of a biopolymer. Alternatively, the viscosity of a specimen solution can be increased by adding to the specimen solution a second polymer other than a polymer to be measured. By the addition of the second polymer, the chain form of the tertiary structure of the biopolymer can be simultaneously straightened. Control of the shape and the speed of movement of the biopolymer is thus enabled. In such a case, preferably a polymer of a size larger than the inside diameter of the nanopore or the width of the channel, more preferably a three-dimensionally cross-linked polymer may be used as the second polymer. This second polymer cannot enter the nanopore, thus enabling exclusion of Raman scattering from the second polymer other than the object to be measured.

Another method for controlling the speed of movement of a biopolymer is a method of applying a pressure difference between the specimen solution existing above the analysis chip and the specimen solution existing below the analysis chip or in the channel. The speed of passage of a biopolymer through the nanopore or the channel can be reduced by applying a force to the biopolymer in the direction opposite to the direction of the force for the biopolymer to pass through the nanopore or the channel based on electrophoresis.

A biopolymer is caused to enter the nanopore or the channel in the analysis chip while the shape and/or the speed of movement of the biopolymer are being controlled. The major axis of the biopolymer is thereby made to coincide generally with the center axis of the nanopore or the channel. As the biopolymer is driven by the specimen moving mechanism, the biopolymer passes through the nanopore or the channel, and unit elements (monomers) constituting the biopolymer pass one after another through the near field formed in the analysis chip. That is, the monomers existing in sequence along the major axis direction of the polymer are successively subjected to the near field, absorb light and produce Raman scattered light. This Raman scattered light is detected and measured with the detection device to successively obtain information on the Raman scattered light derived from the monomers.

In the present invention, characteristics of a biopolymer can be analyzed by using the above-described biopolymer optical analysis apparatus of the present invention. In the present invention, "biopolymer" means, in multimers (oligomers) and polymers in which a plurality of low molecules (monomers) in unit structures bind one to another, those existing in a living body and those derived from those existing in a living body. More specifically, biopolymers include nucleic acids, e.g., a single-stranded DNA (ssDNA), a double-stranded DNA (dsDNA), a single-stranded RNA (ssRNA), a double-stranded RNA (dsRNA) and a hybrid nucleic acid formed of DNA and RNA; peptide nucleic acids; proteins and peptides, e.g., proteins and peptides formed of D- and L-amino acids; sugar chains, e.g., sugar chains in polysaccharides and glycoproteins; and aptamers, e.g., RNA aptamers. Further, biopolymers include polymers containing a sequence or constituent not existing in nature, and polymer molecules artificially synthesized and having, for example, sequences such as poly(A), poly(T), poly(G) and poly(C) or arbitrary sequences. Biopolymers also include nucleic acids prepared by a nucleic acid amplification technique well known in the present technical field (e.g., polymerase chain reaction) and nucleic acids cloned on vectors. A method for preparing specimens including these biopolymers is well known in the present technical field. A person skilled in the art can select a suitable preparation method according to the kind of a biopolymer.

In the present invention, "analysis" designates analysis of characteristics of a biopolymer. In a preferred embodiment, "analysis" designates analysis of the sequential order of monomers existing as units constituting a biopolymer, e.g., analysis of the base sequence in a nucleic acid. To perform analysis of characteristics of a biopolymer, Raman scattered light from each of monomers constituting the biopolymer (from each of bases in a case where the biopolymer is a nucleic acid) is detected and the monomers are qualitatively recognized (identified) based on the results of the detection.

The Raman scattered light is split into transmitted light and reflected light at different ratios for each of wavelengths by the above-described detection device. The intensity of transmitted light and the intensity of reflected light are detected as two-dimensional images by the two-dimensional detector. Characteristics of the biopolymer are analyzed from the ratio of the intensities of the Raman scattered light. That is, the monomers constituting the biopolymer can be qualitatively recognized. Thus, in the present invention, the order in which monomers exist in sequence in a biopolymer can be determined, that is, sequence analysis can be performed by successively obtaining Raman scattered light from the monomers constituting the biopolymer, comparing the results of detection of the obtained Raman scattered light with a reference (standard), determining qualitative characteristics (i.e., kinds) of the monomers, and time-sequentially executing these process steps.

### Embodiments

The present invention will be described more concretely with respect to embodiments thereof with reference to the drawings. However, the present invention is not limited to the embodiments described below.

[Embodiment 1] Nanopore chip of single pore construction and biopolymer characteristic analysis using the nanopore chip

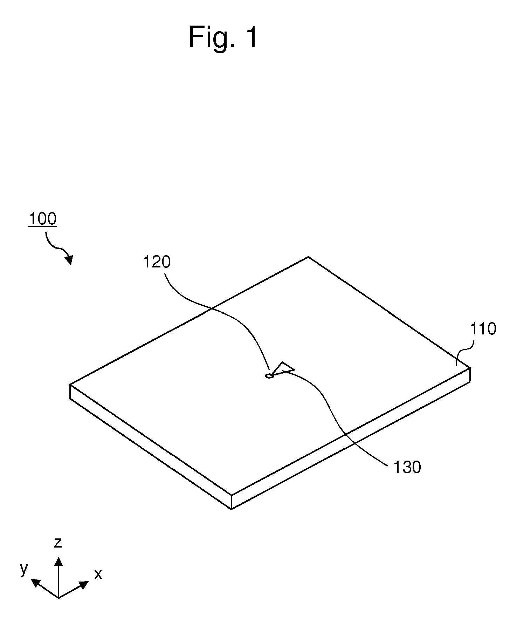

An example of a construction of a nanopore chip for biopolymer characteristic analysis according to the present invention will be described with reference to Figure 1. Figure 1 is a schematic view of a nanopore chip 100. As illustrated, the nanopore chip 100 includes a substrate 100, a nanopore 120 and an electrically conductive thin film 130. As illustrated, a plane parallel to the largest surface of the substrate 110 (hereinafter referred to as "substrate surface") is defined as the xy plane; a direction along which the electrically conductive thin film 130 and the nanopore 120 connect to each other, as x-axis; and a direction perpendicular to the xy plane, as z-axis. The nanopore 120 is formed generally perpendicular to the substrate surface. In other words, a center axis of the nanopore is parallel to the z-axis.

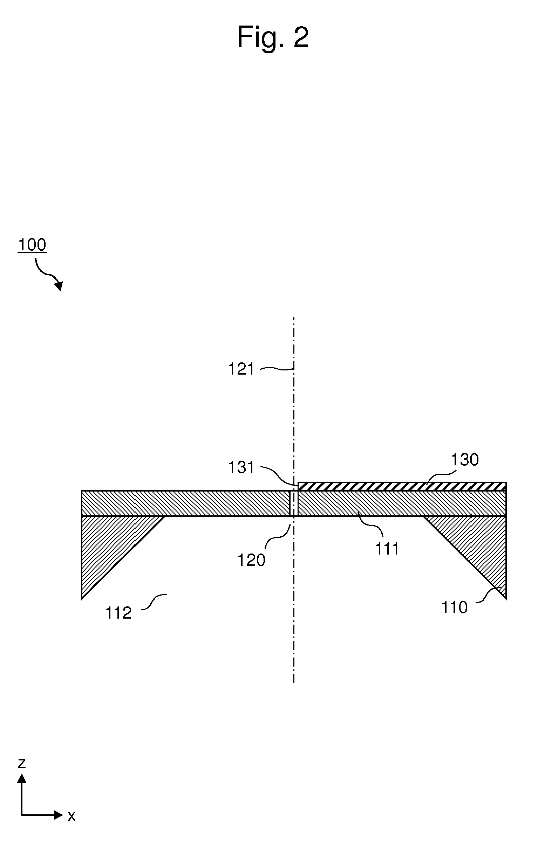

Figure 2 is a schematic enlarged view of the nanopore chip 100 in the present embodiment in a xz section containing the center axis 121 of the nanopore 120. The substrate 110 has a thin film portion 111 on an upper substrate surface as viewed along the z-axis and has the electrically conductive thin film 130 thereabove. The substrate also has a recess in tapered form (hereinafter referred to as "window portion 112") below as viewed along the z-axis. The thin film portion 111 of the substrate is exposed in the window portion 112. The nanopore 120 is formed on the thin film portion 111 of this window portion 112. As illustrated, one end portion 131 of the electrically conductive thin film 130 faces an upper end of the opening portion of the nanopore 120. As roughly illustrated in Figure 1, the end portion 131 is a pointed end as viewed in plan, and this pointed end faces the nanopore 120.

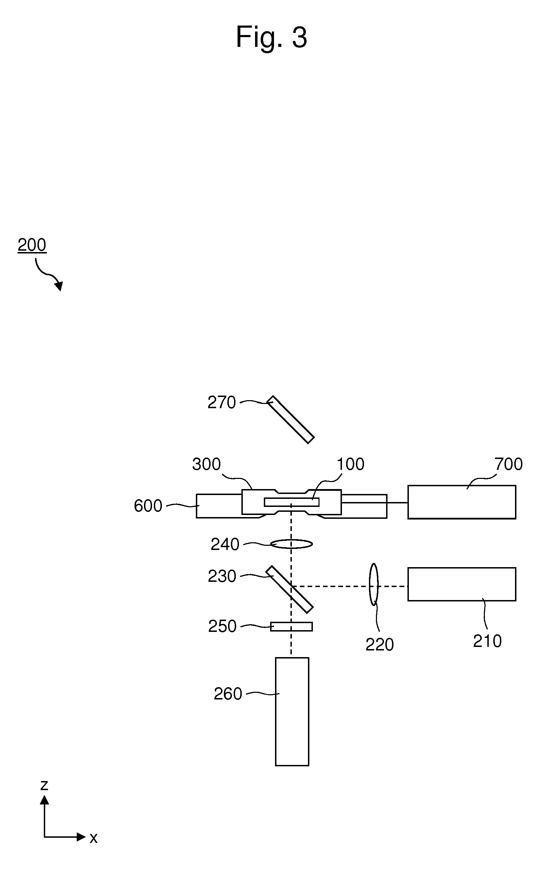

An example of a construction of a biopolymer optical analysis apparatus according to the present invention will next be described with reference to Figure 3. Figure 3 is a schematic construction diagram of a biopolymer optical analysis apparatus 200 in the present embodiment. The analysis apparatus 200 includes a light source 210, a beam expander 220, a dichroic mirror 230, an objective lens 240, a filter 250, a two-split-ratio spectroscopic detection device 260 (described in detail in embodiments 6 and 7), a terminal end 270, an xyz slightly-movable stage 600, a specimen drive device 700, a specimen cell 300, and a measurement control device (not illustrated) such as a personal computer. The nanopore chip 100 is housed in the specimen cell 300.

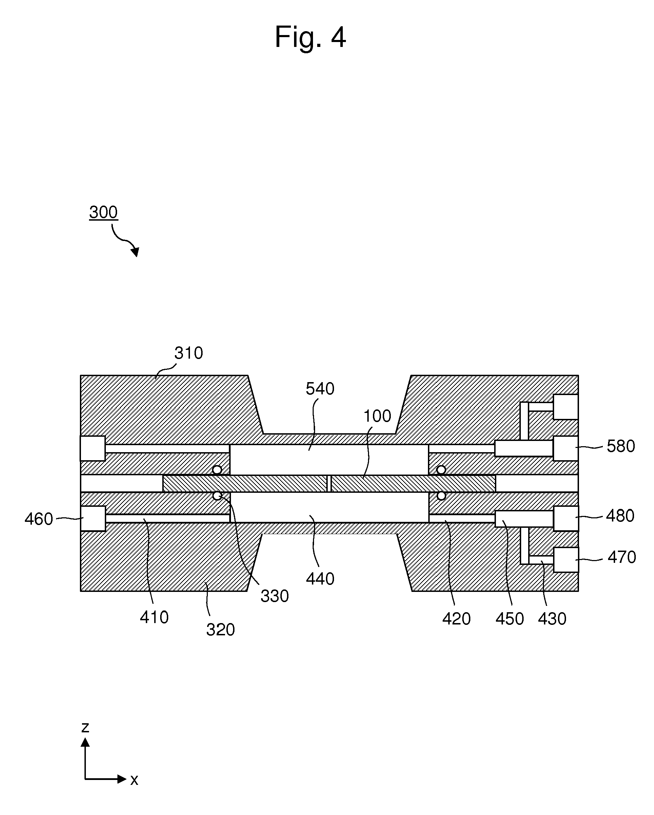

Figure 4 is an xz sectional view schematically showing the construction of the specimen cell 300. The specimen cell 300 includes the nanopore chip 100, an upper member 310, a lower member 320 and screws (not illustrated). In the lower member 320, an O-ring 330, specimen channels 410, 420, and 430, a specimen chamber 440 and an electrode chamber 450 are formed. Specimen connection ports 460 and 470 and an electrode connection port 480 are also formed in the lower member 320. A specimen feed tube (not illustrated) is connected to the lower member 320 through the specimen connection ports 460 and 470 in an airtight manner. A silver-silver chloride electrode (not illustrated) is also connected to the lower member 320 through the electrode connection port 480 in an airtight manner. The silver-silver chloride electrode has silver chloride on its one end, and this end is housed in the electrode chamber 450 (the housed state is not illustrated). The silver-silver chloride electrode has silver on its another end (hereinafter referred to as "silver end"), and this end is exposed outside the electrode connection port 480. The above-described specimen feed tube, specimen connection port 460, specimen channel 410, specimen chamber 440, specimen channel 420, electrode chamber 450, specimen channel 430, specimen connection port 470 and specimen feed tube are filled with a specimen solution (not illustrated) leaving no space therein (without mixing in air bubbles). As a result, the specimen solution in the specimen chamber 440 contacts the silver-silver chloride electrode in the electrode chamber 450, and electrochemical communication is established therebetween. The same description as that for the lower member 320 applies for the upper member 310.

The operation of the nanopore chip according to the present invention will next be outlined with reference to Figures 1 to 8. The specimen cell 300 is first prepared. To be specific, the nanopore chip 100 is interposed between the upper member 310 and the lower member 320 and maintained in pressure contact with the O-rings 330, thereby hermetically forming the upper and lower specimen chambers 540 and 440. A 100 mM KCl aqueous solution is introduced as a specimen solution from the specimen feed tube to fill the specimen chambers 540 and 440 and the electrode chambers 450 with the specimen solution.

Next, the specimen cell 300 is set in the analysis apparatus 200. More specifically, the specimen cell 300 is fixed on the slightly-movable stage 600. The optical system is focused on the thin film portion 111 of the nanopore chip 100 in the specimen cell 300 by using the slightly-movable stage 600 and an optical system for visual inspection not illustrated. Two silver-silver chloride electrodes set on the specimen cell 300 are connected to the specimen drive device 700. The specimen drive device 700 incorporates a voltage source or a current source and can apply a voltage to the lower specimen chamber 440 with respect to the upper specimen chamber 540.

The optical system of the analysis apparatus operates as described below. To be specific, laser light emitted from the light source 210 is shaped by being passed through the beam expander 220 (or a pair of lenses, a beam shaper, or the like), thereafter reflected by the dichroic mirror 230 and condensed on the thin film portion 111 of the nanopore chip 100 by the objective lens 240. The laser light passes through the thin film portion 111 and irradiates the electrically conductive thin film 130 to produce a strong near-field light at the end portion 131 (facing the opening portion of the nanopore 120). When a biopolymer is introduced into the region where this near-field light is formed (hereinafter referred to as "near field"), the near-field light excites the biopolymer to produce Raman scattered light specific to a monomer constituting the biopolymer. The Raman scattered light is condensed by the objective lens 240 and passes through the dichroic mirror 230. Rayleigh scattered light and anti-Stokes lines are removed from the Raman scattered light by the filter 250. Stokes lines in the Raman scattered light are caused to enter the two-split-ratio spectroscopic detection device 260. Differences between transmission and reflection spectra of the Raman scattered light (of Stokes lines) are detected as differences in intensity ratio by using the two-split-ratio spectroscopic detection device 260. The light that has passed through the thin film portion 111 and the electrically conductive thin film 130 is absorbed by the terminal end 270 or dispersed in irrelevant directions.

A procedure of measurement of DNA as an example of a biopolymer to be measured is as described below. That is, a specimen prepared by dissolving, for example, a single-stranded DNA having a length of 10 kb (knt) in a 100 mM KCl aqueous solution so that the concentration of the DNA was 1 nM was used. This solution is introduced as a lower specimen solution into the specimen chamber 440. When a negative voltage of 100 mV is applied to the lower specimen chamber 440 by using the specimen drive device 700, ions in the specimen solution is electrophoresed through the nanopore 120 and a current (ion current) flows. Since only water and KCl exist in the near field at first, Raman scattered light from water only is observed. The DNA is electrophoresed from the lower specimen chamber 440 into the upper specimen chamber 540 through the nanopore 120. When the DNA passes through the nanopore 120, a nucleic acid base that is a constituent of the DNA enters the near field formed at the end portion 131 of the electrically conductive thin film 130. Raman scattered light specific to the base is then produced and the intensity ratio based on the Raman scattering spectrum is obtained by the two-split-ratio spectroscopic detection device 260. As DNA electrophoresis is continued, the nucleic acid base moves and comes off the near field. Then the Raman scattered light specific to the base becomes extinct. As electrophoresis is further continued, the process in which the next base in the DNA sequence also enters and comes off the near field is repeated, thus obtaining, with time, changes in intensity ratio based on Raman scattering spectra corresponding to the sequence of bases. A design to set different intensity ratios for each of the bases is made. A signal for the intensity ratio is obtained with time and the base sequence of the DNA is obtained from changes in the signal. The foregoing is an outline of the operation in the present embodiment.

Each component will be described in detail below.

The nanopore chip 100 in the present embodiment was made by a process described below. A silicon substrate was used as substrate 110 and an oxide film having a thickness of about 20 nm was formed on a surface of the substrate by low-pressure chemical vapor deposition (LPCVD) (this oxide film finally becomes the thin film portion 111). The window portion 112 with the thin film portion 111 was formed by forming a pattern for the window portion at the substrate bottom by electron beam (EB) lithography, removing a surface layer by reactive ion etching, and thereafter removing silicon by KOH (potassium hydroxide) wet etching. Silver thin film was formed as electrically conductive thin film 130 on the substrate surface by sputtering. The thickness of the silver film was set to about 5 nm. A resist was applied on the silver thin film, and a pattern similar to the triangular shape shown in Figure 1 was formed by EB lithography. Silver in the region other than the triangular pattern was removed by etching and the resist was removed. Finally, the nanopore 120 was formed by irradiating the end portion 131 of the triangular shape with a TEM electron beam while observing the substrate with a transmission electron microscope (TEM). The inside diameter of the nanopore was about 10 nm. While the thin film portion 111 was formed by using silicon oxide in the present embodiment, silicon nitride or the like can be used in the same way.

While silver was used as the material of the electrically conductive thin film 130 in the present embodiment, this material is not limited to silver. Any of general materials may be used if it has electrical conductivity. In ordinary cases, a metal can be preferably used. Examples of other metals usable as electrically conductive thin film are platinum group metals, such as platinum, palladium, rhodium and ruthenium, gold and copper.

In the present embodiment, a transparent member was used for the specimen cell 300, particularly for central portions of the upper member 310 and the lower member 320 (portions that isolates the specimen chambers 440 and 540 from the outside). Examples of a transparent member usable therefor are glass, quartz and a plastic material such as acrylic having high transparency at the light source wavelength. Use of a transparent member for the central portion of the lower member 320 enables efficient application of laser light from the light source 210 to the nanopore chip 100. Also, use of a transparent member for the central portion of the upper member 310 enables transmitted light and scattered light having passed through the nanopore chip 100 to pass without being reflected so that background light is reduced.

In Figures 3 and 4, the objective lens 240 and the specimen cell 300 (the nanopore chip 100 contained in the specimen cell 300) are illustrated at some distance from each other. In the actual apparatus, however, it is desirable to place these two components as close as possible to each other. It is desirable to set the distance between the objective lens 240 and the nanopore chip 100 as small as 3 mm or less, preferably 1 mm or less. In this way, the efficiency of excitation by the excitation light and the efficiency of collection of scattered light can be improved to perform measurement with high sensitivity. A liquid immersion type of lens is preferably used as objective lens 240. Preferably, the objective lens is of a high-aperture type. A numerical aperture ≥ 0.8 is particularly preferred.

The biopolymer optical analysis apparatus in the present embodiment was constructed based on a microscope integrated laser Raman spectroscopic apparatus similar to one based on the third related art. However, a stage attached to a microscope was used as xyz slightly-movable stage 600; a piezo-stage for AFM was not used. In the present embodiment, a Kr ion laser having an output of 1 mW and a wavelength of 531 nm was used as light source 210. An arbitrary function generator was used as specimen drive device 700. The arbitrary function generator was used in combination with an attenuator (resistor voltage divider) as required. The specimen drive device 700 can output a DC or an arbitrary waveform in an output voltage range of 0 to ±10V. As a typical example of the arbitrary waveform, a pulse wave may be mentioned. The time width of peaks of pulses is set in steps of 10 nanoseconds and the value of the pulse peak voltage can be arbitrarily output in the above-mentioned output voltage range.

The operation in the present embodiment will be described below in detail.

The substrate 110 of the nanopore chip 100 is formed mainly of silicon (Si). The thickness of the substrate is about 700 µm. The thin film portion 111 of the substrate is formed of silicon oxide (SiO₂) and the thickness thereof is as thin as about 20 nm. In the window portion 112 of the substrate, therefore, laser light passes through the thin film portion 111 to irradiate the electrically conductive thin film 130. By irradiation of the electrically conductive thin film 130 with laser light, a strong near field is generated at the end portion 131. The thickness of this near field in the z-direction is approximately equal to the thickness of the electrically conductive thin film 130, i.e., about 5 to 10 nm.

In the portion other than the window portion 112, the substrate 110 has a thickness of about 700 µm. Since Si as the material of the substrate 110 absorbs, reflects and scatters laser light, substantially no laser light reaches the thin film portion 111 formed above the region other than the window portion 112 and the electrically conductive thin film 130 formed on this thin film portion 111. Therefore, the formation of the near field on the electrically conductive thin film 130 is inhibited on the region other than the window portion 112. That is, advantageously, the above-described construction enables restricting the formation of the near field on the electrically conductive thin film 130 mainly to the region of the window portion 112, particularly the end portion 131 where the near field is to be formed, and inhibiting the generation of background light in the region other than the region where the near field is to be formed.

The results of analyzing and comparing the formations of near fields through a simulation with respect to the structure adopted in the present embodiment and a structure adopted in a modified example described later will be described below.

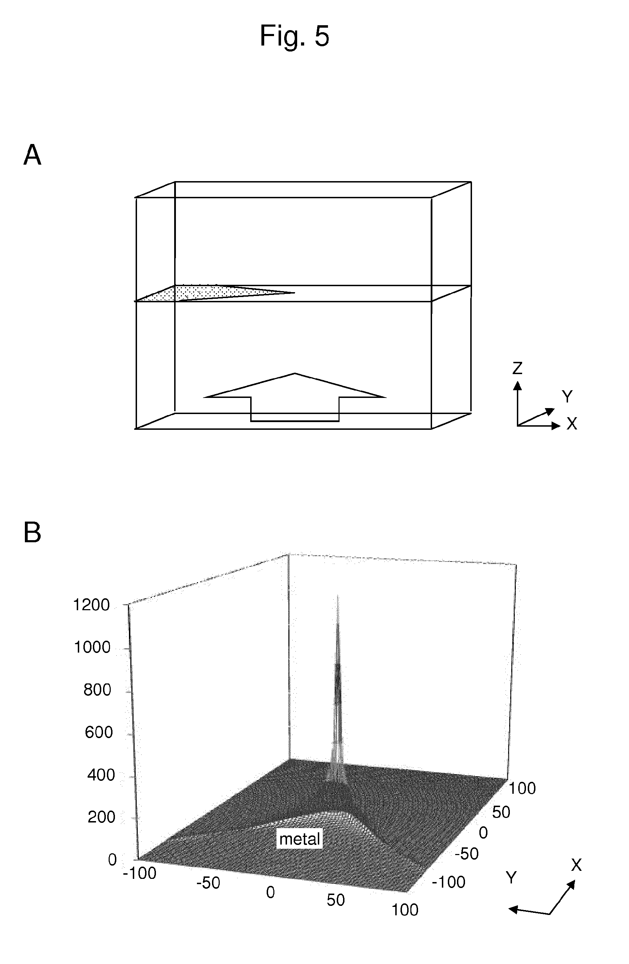

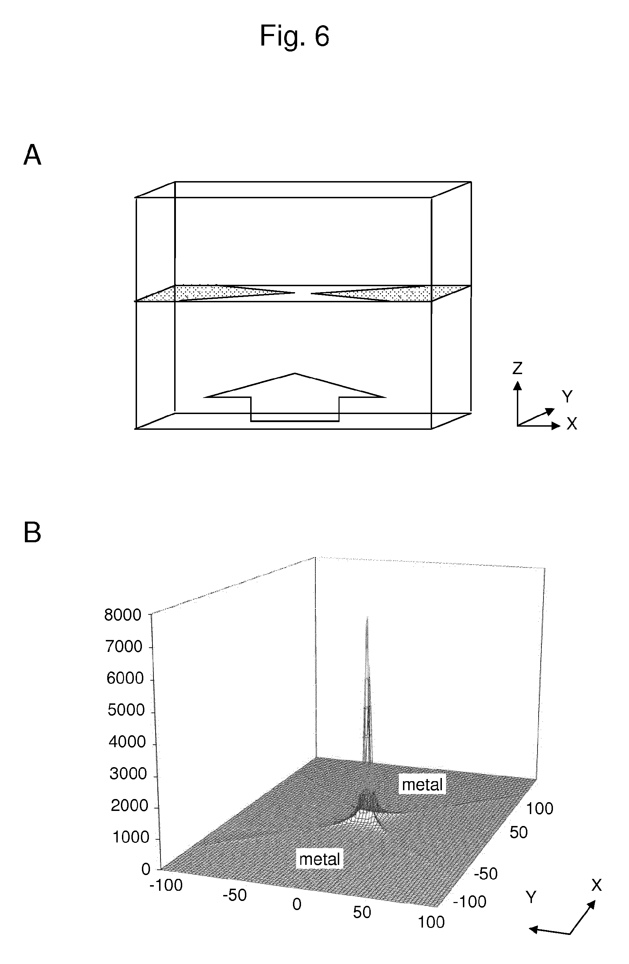

A distribution of near-field light produced in the vicinity of a triangular conductor when light was incident on the conductor, as in the present embodiment, was calculated by using an FTDT method (a time-domain light propagation solver OptiFDTD, Optiwave System Inc.). In this calculation, the size of the analysis region was set to 0.3 x 0.2 x 2.6 µm in the X-, Y-, and Z-directions (X, Y, Z form a coordinate system used only in Figures 5 and 6, and Y is the direction perpendicular to the XZ plane). The material of the conductor was assumed to be silver, the thickness was set to 10 nm and the tip point angle was assumed to be 90 degrees. The incident wave was assumed to be a plane wave having a wavelength of 780 nm, and a wave source was generated at a position one wavelength apart from the surface of the conductor (λ= 780 nm). The direction of polarization of the incident wave was assumed to be the X-direction. A periodic boundary condition was set with respect to the XY side surface, while an absorption boundary condition was set with respect to the Z-direction side surface. The mesh size was uniformly set to 2.6 nm through the entire calculation region.

A of Figure 5 is a schematic diagram of the analyzed structure. In B of Figure 5, an XY plane distribution of the ratio of the result of calculation of the near field intensity density (I) and the intensity density (Iin) of the incident wave was plotted on the ordinate. As shown in the diagram, the strongest field of light occurred at the tip of the thin film triangle, the maximum value of the field was about 1100 times larger than the incident intensity ratio.

Calculation was also performed for a case where two corresponding triangular pieces of thin film were provided and opposed to each other with their apexes being spaced apart by 3 nm. The results of this calculation are shown in A and B of Figure 6. This corresponds to the modified example described later. In this case, an effect of about 7100-times enhancement was obtained.

In summary, the results of the above-described simulation suggest that a near field having a strong photoelectric field enhancement effect is formed by using particularly an electrically conductive thin film provided in a structure and a shape similar to those in the present embodiment or the modified example described later.

When Raman scattered light derived from a base is observed immediately after DNA enters the nanopore 120, the electrophoresis speed of the DNA can be reduced by reducing the absolute value of the voltage applied to the lower specimen chamber 440 by using the specimen drive device 700. Also, the DNA chain can be electrophoresed at a low speed in the reverse direction by inverting the polarity of the applied voltage (setting the voltage on the lower specimen chamber 440 positive) in a state where the absolute value of the voltage is reduced. By continuing electrophoresis under this condition until a state occurs where no Raman scattered light derived from the base is observed, the leading end of the DNA chain can be forced back out of the near field. The DNA chain can be slowly electrophoresed from its leading end by resetting the polarity of the applied voltage (setting the voltage on the lower specimen chamber 440 negative) while the absolute value of the voltage is maintained at the reduced level, thus enabling repeating Raman scattering measurement. Thus, the base to be measured can be made to stay in the near field for a time period necessary for measuring a Raman scattering spectrum with sufficiently high accuracy.

The voltage applied to the lower specimen chamber 440 by using the specimen drive device 700 can be made constant (DC). It is also possible to repeat electrophoresing DNA and stopping electrophoresing in a short cycle by using pulse waves. In this case, the pulse width of the pulse waves can be adjusted (pulse width modulation). It is possible to reduce the electrophoresis time in one cycle and increase the stoppage time in the one cycle by reducing the proportion (duty ratio) of the time period during which the pulse is ON in the one cycle and increasing the proportion of the time period during which the pulse is OFF in the one cycle. For example, since the pulse width can be changed in steps of 10 ns by using the arbitrary function generator having a 100 MHz frequency range for example, the duty ratio can be adjusted with a resolution of 1/1,000,000 when the length of one cycle is set to 10 ms for example. That is, the average DNA chain movement speed can be adjusted (low) according to the duty ratio with extremely high resolution. Combination with pulse wave height adjustment (pulse height modulation) enables further accurate adjustment of the electrophoresis speed. Control of the electrophoresis voltage and the pulse width of the voltage enables making the base to be measured stay in the near field for a time period necessary for measuring a Raman scattering spectrum with sufficiently high accuracy. Also, an undesirable state where the signal varies as the object to be measured enters the near field and comes off the near field during measurement can be avoided by obtaining a Raman scattering spectrum in the stoppage time to improve the accuracy of the measured value.

It is also possible to periodically change the voltage to be applied to the lower specimen chamber 440 between a positive level and a negative level. In doing so, the voltage may be adjusted so that the time averaged voltage is a somewhat negative voltage, thereby stably extending a DNA chain and slowly electrophoresing the DNA chain into the upper specimen chamber 540 through the nanopore 120 in comparison with the case of simply maintaining the voltage at a negative level, thus enabling measurement of Raman scattering based on each base in the DNA chain with improved sensitivity.

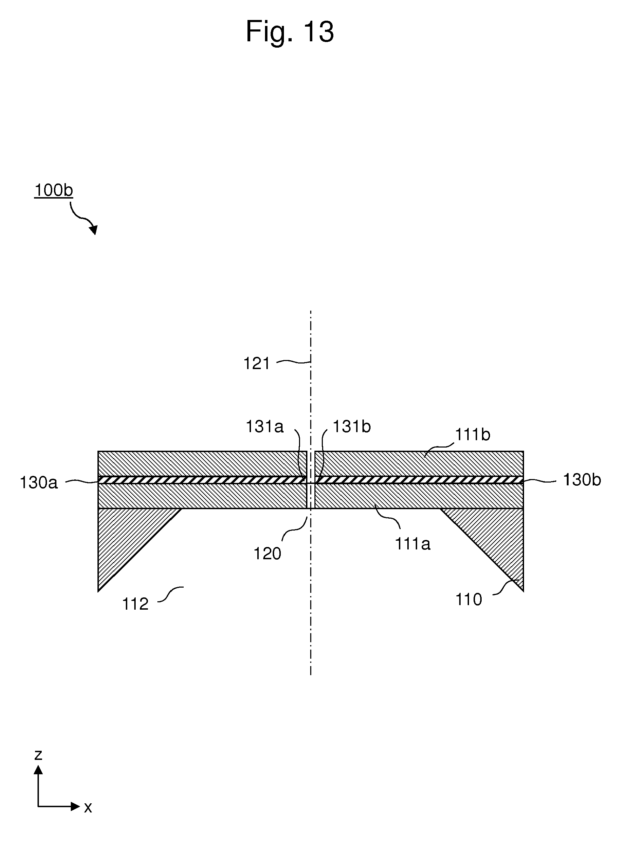

As another means for controlling the DNA chain electrophoresis speed, increasing the viscosity of the specimen solution is effective. If a mechanism for controlling the temperature around the nanopore chip is added to reduce the temperature of the specimen solution, the viscosity of the specimen solution can be increased and the Brownian movement of a DNA chain can be limited. This is, therefore, a good condition for DNA chain Raman scattering measurement. Also, addition of a polymer other than the object to be measured to the specimen solution enables straightening the chain form of the tertiary structure of a DNA chain as well as increasing the viscosity of the specimen solution. This is, therefore, a good condition for DNA chain Raman scattering measurement. As this polymer, a separation medium for capillary electrophoresis may be used. Use of a polymer, preferably a polymer larger than the inside diameter of the nanopore, more preferably a three-dimensionally cross-linked polymer enables increasing only the viscosity without interference with the measurement. In particular, referring to Figure 13 for an embodiment described later, the enhancement field, i.e., the measurement region, can be confined in the nanopore. In this case, only a biopolymer to be measured, e.g., a DNA chain can be caused to enter the measurement region, while polymers other than the object to be measured are not allowed to enter the measurement region, thus enabling exclusion of Raman scattering from polymers other than the object to be measured.

For the purpose of reducing the DNA electrophoresis speed, other methods are adoptable as a method for realizing an extremely low electrophoresis speed and for accurately controlling electrophoresis at such a speed. According to a first one of such methods, a pair of measuring electrodes for detecting the voltage between the upper and lower specimen chambers (440 and 540) with high sensitivity may be newly provided respectively in the upper and lower specimen chambers (separately from the existing silver-silver chloride electrodes). In this case, a predetermined extremely low voltage can be applied with accuracy between the upper and lower specimen chambers by performing feedback control of the voltage applied to the silver-silver chloride electrodes based on the actual voltage measured by using the measuring electrodes. As a second method, a potentiostat method is adoptable. In this case, the existing pair of silver-silver chloride electrodes are regarded as a specimen electrode and a counter electrode, a reference electrode is newly provided in the specimen chamber 440 and 540 on the counter electrode side, and a current flowing between the specimen electrode and the counter electrode can be controlled so that the voltage between the specimen electrode and the reference electrode becomes equal to a set value. Each of the above-described methods enables application of a predetermined extremely low voltage between the upper and lower specimen chambers 440 and 540 with accuracy, realizes an extremely low electrophoresis speed and enables accurate control of the electrophoresis speed. As a third method, a galvanostat method is adoptable. In this case, the existing pair of silver-silver chloride electrodes are regarded as a specimen electrode and a counter electrode, and feedback control can be performed while monitoring the current flowing from one of the electrodes to the other so that the current becomes equal to a predetermined value. This method enables causing a predetermined extremely low current to flow between the upper and lower specimen chambers 440 and 540 with accuracy, realizes an extremely low electrophoresis speed and enables accurate control of the electrophoresis speed.

A still another method for controlling the speed of passage of a DNA chain through the nanopore 120 will be described. A pressure difference is produced between the specimen solution filling the lower specimen chamber 440 and the specimen solution filling the upper specimen chamber 540 to apply a force to a DNA chain in the direction opposite to the direction of the force for the DNA chain to pass through the nanopore based on electrophoresis. The speed of passage of a DNA chain through the nanopore can be reduced in this way. For example, a pressure equal to or higher than the atmospheric pressure is applied to the specimen solution filling the upper specimen chamber 540 by a pump mechanism or a piezo mechanism, while the specimen solution filling the lower specimen chamber 440 is maintained at the atmospheric pressure, thereby enabling generation of a pressure in a direction from the upper specimen chamber 540 toward the lower specimen chamber 440, i.e., the direction opposite to that of DNA chain electrophoresis. This pressure difference may be controlled with an osmotic pressure based on a difference between the compositions of the specimen solutions in the upper and lower sections, e.g., a difference in ion concentration. By this pressure difference, the specimen solution may be caused to flow in the nanopore 120 as a bulk in the direction opposite to the direction of DNA chain electrophoresis, i.e., the direction from the upper specimen chamber 540 toward the lower specimen chamber 440 to reduce the DNA chain passage speed. This specimen solution flow can also be realized by providing electric charge on the inner surface of the nanopore 120 so that an electroosmotic flow is caused in the nanopore 120. By the specimen solution flow, an effect described below can also be produced. The specimen solution can flow in such a manner as to wrap around a DNA chain so that the DNA chain is maintained in a local region about the center axis of the nanopore 120 in the nanopore 120 and is extended along the center axis direction. This enables each base of the DNA chain to pass through the center of the enhancement field with stability, thus realizing high-accuracy Raman scattering measurement.

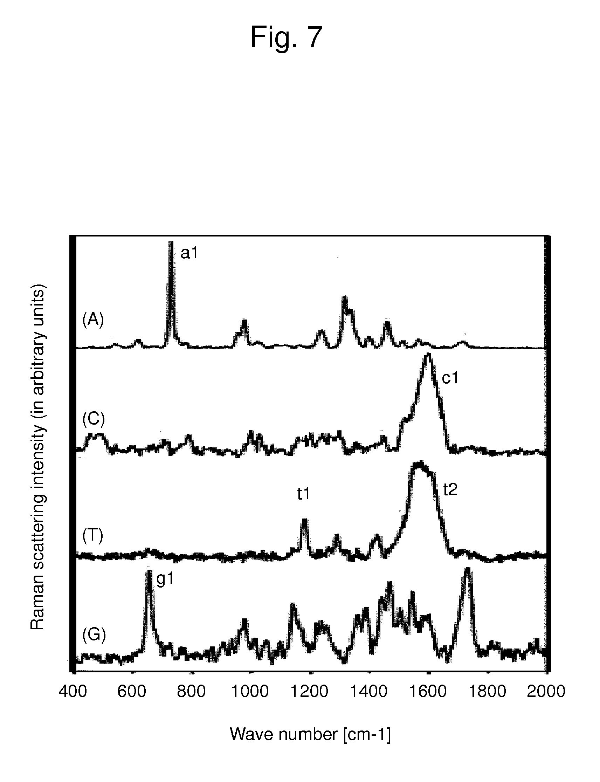

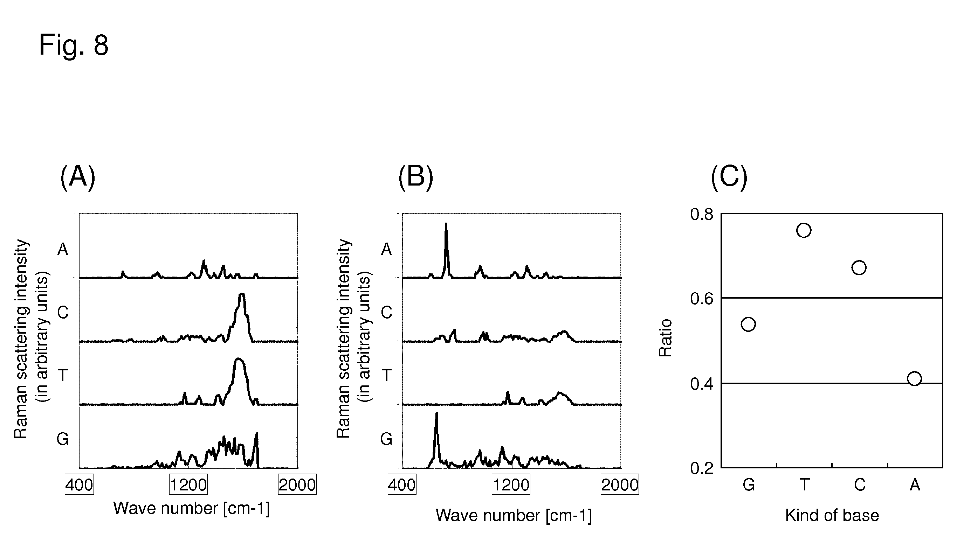

Figure 7 shows an example of a typical Raman scattering intensity (spectrum) of nucleic acid bases. Four kinds of bases A, C, T, and G respectively exhibit peaks of scattered light intensity at characteristic wave numbers (also referred to as "characteristic band" below). In this example, the peaks in characteristic bands of A, T, and G are respectively indicated by a1, t1, and g1, and the corresponding wave numbers are about 730 cm⁻¹, 1180 cm⁻¹, and 650 cm⁻¹, respectively. A peak position c1 (i.e., about 1730 cm⁻¹) of C overlaps a peak position t2 (i.e., 1600 cm⁻¹) of T to a certain extent. However, T and C can be identified by considering the existence/nonexistence of the characteristic band t1 specific to T. There is also a possibility of C: 1260 cm⁻¹ and T: 1360 cm⁻¹ being applicable as characteristic bands of C and T other than those described above. In the case of identifying and detecting peaks themselves in this way, there is a need to detect Raman scattering spectra with a resolution of 10 cm⁻¹ for example. That is, there is a need to divide the frequency range from 400 cm⁻¹ to 1800 cm⁻¹ by 140 per nanopore for detection. Therefore a large number of pixels in the detector are required. Because of the need for a large number of pixels, measurement cannot be suitably performed in a case where high-speed measurement is demanded. Detection of differences between spectra of Raman scattered light as differences in intensity ratio with a two-split-ratio spectroscopic detection device, as in the present embodiment, enables detection and identification basically with signals for two pixels per nanopore, thus enabling cost reduction by reducing the number of pixels of the detector and enabling high-speed repetitive measurement. More specifically, as shown in Figure 8, Raman scattered light is split into transmitted light and reflected light at different ratios in a particular wavelength range, and the four kinds of bases can be identified from the intensity ratios between the transmitted light and the reflected light (related details will be described with respect to Embodiment 6).

In the present embodiment, examples of analysis of DNA by obtaining spectra from A, C, T, and G have been described. However, the range of application of the present invention is not limited to this. For example, analysis of RNA can be performed by analyzing a spectrum from U. Also, direct read of methylation of DNA is enabled by obtaining a spectrum from methylated C. Further, analysis of a peptide or a protein is enabled by obtaining spectra from amino acids, and analysis of a sugar chain is enabled by obtaining spectra from saccharides.

The biopolymer optical analysis apparatus according to the present embodiment is based on a solid-state nanopore and therefore has the advantage of having high structural stability and high reliability. In the present embodiment, the analysis apparatus determines kinds of monomers by referring to Raman spectra. With respect to spectrum measurement, the analysis apparatus has intensity ratios and intensities as two-dimensional information and therefore has the advantage of having a much larger amount of information than in the case of having one-dimensional information such as tunnel current intensity, high qualitative discriminability and, hence, high base discriminability. In the present embodiment, a near field is fixed at the opening portion of the nanopore 120, DNA is electrophoresed by using the specimen drive device 700 to control the relative positional relationship with the near field. Therefore, there is no need to fix a nucleic acid on the solid substrate 110 in advance, nor to provide a high-resolution slightly-movable stage and an AFM. Also there is no need for a delicate operation to perform two-dimensional scanning with an AFM probe with sub-nm accuracy. That is, the analysis apparatus has the advantage of being simple in construction and operation.

### [Modified example of Embodiment 1]

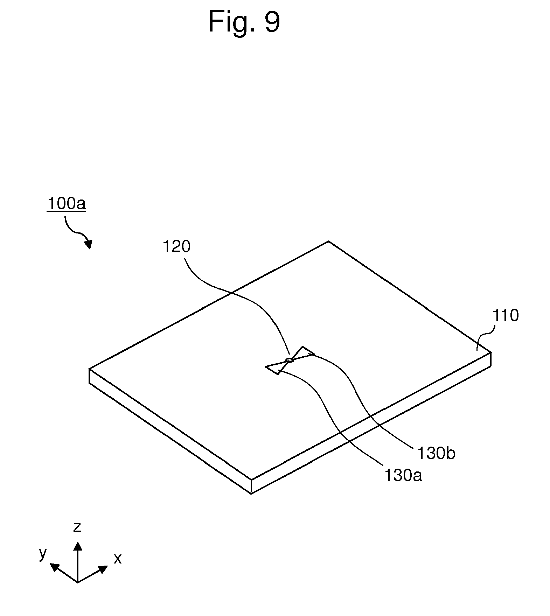

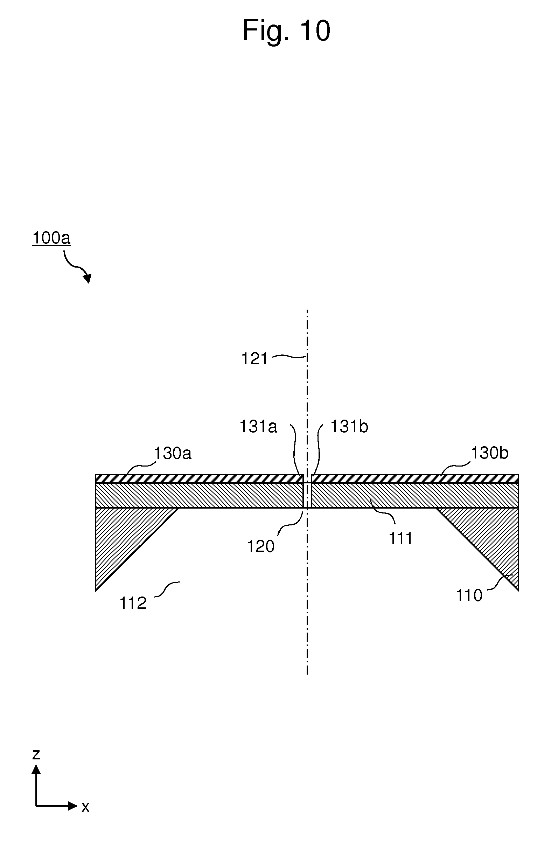

A nanopore chip 100a of a construction described below can be implemented as a modified example of embodiment 1. Figure 9 is a schematic view of the nanopore chip 100a according to the present modified example. As illustrated, the nanopore chip 100a includes a substrate 110, a nanopore 120 and electrically conductive thin films 130a and 130b. That is, the present embodiment differs from embodiment 1 (having only one electrically conductive thin film 130) in that two electrically conductive thin films corresponding to the electrically conductive thin film 130 in embodiment 1 are provided. Figure 10 is a schematic enlarged view of an xz section containing a center axis 121 of the nanopore 120 in the nanopore chip 100a in the present modified example. As illustrated, the electrically conductive thin films 130a and 130b are formed above a thin film portion 111 as viewed along the z-axis, and end portions 131a and 131b of the thin films each face an upper end of an opening portion of the nanopore 120 and are opposed to each other. That is, the electrically conductive thin films 130a and 130b are disposed by being spaced apart from each other by a distance substantially equal to the opening diameter of the nanopore 120.

The analysis apparatus and the operation for the nanopore chip 100a according to the present modified example are similar to those in embodiment 1. Differences therebetween reside in that near field light produced by irradiation with laser light is produced at the gap between the end portions 131a and 131b of the electrically conductive thin films 130a and 130b. Also, near field lights derived from the two electrically conductive thin films intensify each other so that the intensity of the near field is enhanced, as suggested by the simulation results shown in embodiment 1. Also, because of the existence of the electrically conductive thin films 130a and 130b, the distribution of the near field along the x-direction is restricted and localized to about the opening diameter of the nanopore 120. As a result, the degree of symmetry in the shape of the near field in the present modified example is high and, therefore, the degree of uniformity thereof is high. In the present modified example, the intensity of the near field is as high as about 7000 times the quantity of incident of light, as described above (Figure 6). Therefore, advantageously, the sensitivity is high, the near field is spatially uniform, and the spatial resolution is also high.

### [Embodiment 2] Construction of multi-nanopore analysis apparatus

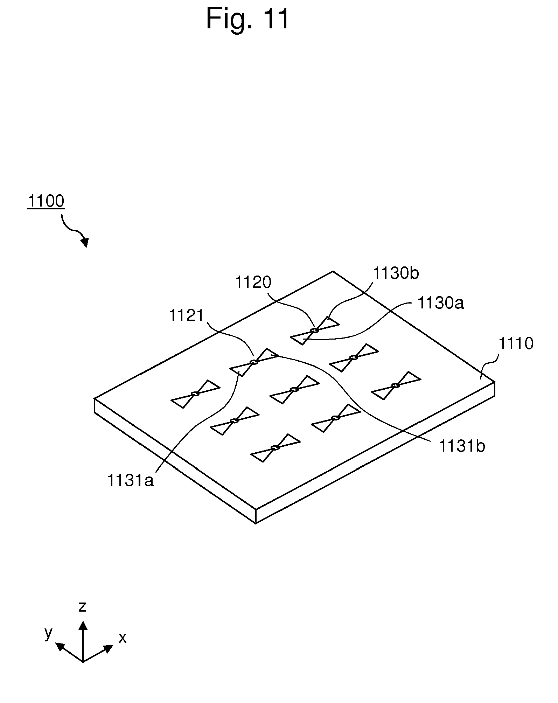

An example of a construction of a multi-nanopore chip for biopolymer characteristic analysis according to the present invention will be described with reference to Figure 11. Figure 11 is a schematic view of a multi-nanopore chip 1100 in the present embodiment 2. As illustrated, the multi-nanopore chip 1100 includes a substrate 1110, nanopores 1120 and 1121 and electrically conductive thin films 1130a, 1130b, 1131a, and 1131b. As illustrated, the multi-nanopore chip 1100 in the present embodiment has on one substrate 1110 a plurality of unit structures each of which corresponds to the unit structure in the above-described modified example including a nanopore and opposed electrically conductive thin films, i.e., the unit structure illustrated in Figure 10.

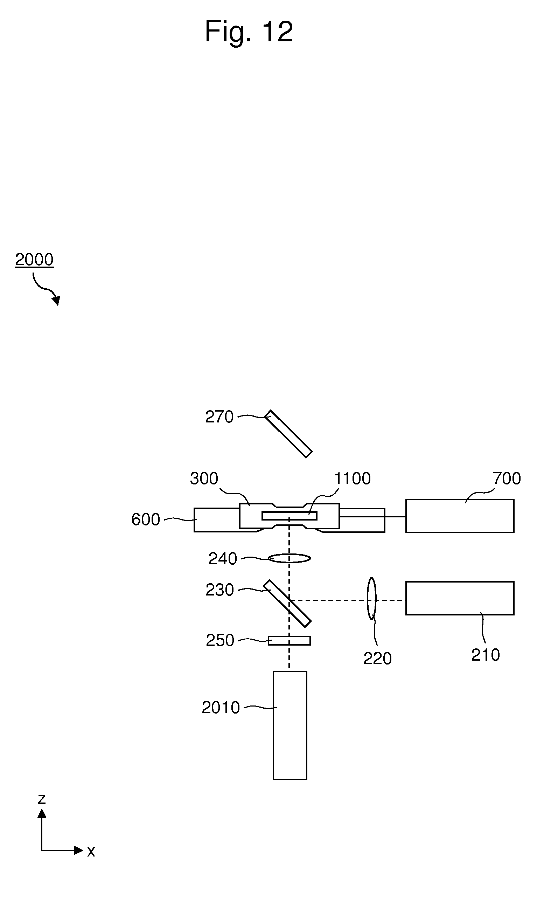

An example of a construction of a biopolymer optical analysis apparatus in the present embodiment will next be described with reference to Figure 12. Figure 12 is a schematic construction diagram of a multi-analysis apparatus 2000 that performs biopolymer characteristic analysis in the present embodiment 2. The multi-analysis apparatus 2000 includes a light source 210, a beam expander 220, a dichroic mirror 230, an objective lens 240, a filter 250, a two-split-ratio spectroscopic detection device 2010, a terminal end 270, an xyz slightly-movable stage 600, a specimen drive device 700, a specimen cell 300, and a measurement control device (not illustrated) such as a personal computer. The multi-nanopore chip 1100 is housed in the specimen cell 300.

The outline of operation in the present embodiment will next be described. The operation in the present embodiment 2 is the same as that in embodiment 1 but the multi-nanopore chip 1100 having a plurality of unit structures as a nanopore chip is used. Accordingly, Raman scattered lights derived from a specimen are respectively produced independently in a plurality of places on the multi-nanopore chip 1100. The Raman scattered lights are condensed by the objective lens 240 and pass through the dichroic mirror 230. After Rayleigh scattered light has been removed by the filter 250, the Raman scattered lights form images on the detection surface of the two-split-ratio spectroscopic detection device 2010. The Raman scattered lights from the individual nanopore opening portions are detected as two-split images such that their intensities are split at different ratios for each kind of polymer, thus enabling Raman scattered lights from the multi-nanopore to be simultaneously detected and discriminated. In the case of the conventional system in which Raman scattered light is detected by being wavelength-dispersed, 140 pixels or more are required per nanopore, as described in embodiment 1. In a case where the pixel size of the two-dimensional detector is 8 microns and the image-forming magnification is 60x, overlaps occur between spectra and it is difficult to perform measurement when the distance between each adjacent pair of nanopores on the substrate is about 20 microns. In the present invention, since Raman scattering can be measured as images of bright spots without being wavelength-dispersed, measurement can be performed by avoiding overlap of a dispersion image from each adjacently-disposed nanopore. Therefore, the nanopores can be disposed by being spaced one from another by a small distance on the multi-nanopore chip. For example, the nanopores can be disposed like lattice points of a square lattice of about 0.5 to several microns, thus enabling high-density disposition. In the case of Raman scattering measurement in particular, the field size is reduced because of detection with a high-resolution high-NA lens. High-density disposition is made possible, as in the present embodiment, thus enabling measurements on a larger number of nanopores and improving the throughput of analysis.

In the case of performing wavelength dispersion, the set position of the multi-nanopore chip 1100 with respect to the optical system center axis can vary measurement by measurement, and there is, therefore, a need to perform a calibration of the relationship between the pixel coordinates and the wavelength, i.e., wavelength calibration, from the position on the detection surface for each nanopore each time a measurement is made. In the present embodiment, however, since only detection of images of bright spots suffices, there is no need for a wavelength calibration operation or the like, and measurement can be performed in a simple way.

A DNA chain passing through the nanopore by electrophoresis ordinarily moves at a high speed. For example, when a voltage of 100 mV is applied, the time required for nanopore passage of a single-stranded DNA having a base length of 10 kb (knt) is about 1 ms, and the enhancement field stay time per base is only 0.1 µs (assuming that the spatial stretch of the enhancement field is the infinitesimal). Therefore, independently measuring Raman scattering signals from bases under this condition requires setting the operating speed of the two-split-ratio spectroscopic detection device 2010 to 1 MHz or higher. In a case where fluorescence, phosphorescence, scattered light or the like is measured with high sensitivity by a conventional microscopic system, particularly when two-dimensionally distributed objects to be measured are simultaneously measured, the operating speed of the detector is ordinarily 30 Hz or lower and is less than 1 kHz when it is particularly high. Therefore, a new challenge as a result of combining nanopores and Raman scattering spectrum measurement on DNA chains passing through the nanopores in the present invention is to set the operating speed of the detector to 1 kHz or higher, preferably 1 MHz. As a means for realizing such high-speed detection, a complementary metal-oxide-semiconductor (CMOS) is preferred rather than a charge-coupled device (CCD) ordinarily used in conventional microscopic systems. While a CCD makes A/D conversion on the entire detection area or on pixels sequentially on a column-by-column basis, a CMOS can simultaneously make A/D conversion on all detection pixels two-dimensionally arrayed and is capable of reducing the time required for A/D conversion to a several hundredth to several thousandth. With any detector, not limited to CMOS, the same effect can be obtained if the detector has a function to make A/D conversion on each detection pixel. In reducing the time required to transfer a large amount of signals obtained by the detector to a control computer through a cable and a board and to write the signals to an incorporated hard disk or the like, it is effective to incorporate a large-capacity memory in the detector and store the large amount of signals in the memory through a line not routed via the above-described devices. On the other hand, with speed-up of detection, the exposure time in each measurement is considerably reduced. An arrangement for preventing a reduction in sensitivity due to this cause, for example, by introducing an enhancement field, using a high-aperture objective lens of a liquid immersion type, using a high-sensitivity element such as an avalanche photodiode or the like as the detector, or providing a multiplying function of an image intensifier, an electron multiplier CCD (EM-CCD), or the like is preferable for the present invention. That is, it is preferable to use a detector having a multiplication mechanism in the present invention.

According to the present embodiment 2, Raman scattering spectra can be simultaneously obtained with respect a plurality of nanopores. Therefore, advantageously, the degree of multiplexing of measurements is high and the reliability of measurement results is high. Also, the throughput in the case of performing measurement with a high degree of multiplexing is high.

A modified example of the present embodiment is conceivable in which independent specimen chambers are provided for each individual nanopore and different specimens are simultaneously measured by using the nanopores. Advantageously, in this modified example, measurements can be made on a plurality of specimens in parallel with each other and, therefore, the throughput is high.

In the arrangement according to the present embodiment, a nanopore is formed between a pair of electrically conductive thin films (e.g., 1130a and 1130b, or 1131a and 1131b) and a biopolymer passes through the nanopore. A similar arrangement can also be implemented by providing a channel between the pair of electrically conductive thin films in place of the nanopore. A biopolymer is caused to move parallel to the substrate surface to pass through the gap between each pair of electrically conductive thin films (e.g., 1130a and 1130b, or 1131a and 1131b), and Raman scattered light then produced is detected in the same way, thus enabling parallel measurements on the plurality of specimens and, hence, high-throughput analysis.

### [Embodiment 3] Nanopore chip of sandwich structure

An example of a construction of a nanopore chip for performing biopolymer characteristic analysis according to the present invention will be described with reference to Figure 13. Figure 13 is a schematic enlarged view of an xz section containing a center axis 121 of the nanopore 120 in the nanopore chip 100b in the present embodiment 3. A substrate 110 has a thin film portion 111a on an upper substrate surface as viewed along the z-axis, electrically conductive thin films 130a and 130b on the thin film portion 111a, and a thin film portion 111b on the electrically conductive thin films 130a and 130b. In other respects, the construction is the same as that in the modified example (Figure 10) of embodiment 1.

A method of making the nanopore chip 100b in the present embodiment 3 is similar to that in (the modified example of) embodiment 1, but differs in that after a pattern for the electrically conductive thin films 130a and 130b has been formed by EB lithography, the thin film portion 111b having a thickness of about 20 nm and formed of SiO₂ is formed by sputtering and the nanopore is thereafter formed by TEM. Since the thin film portion 111b is thin, the appearance of the nanopore chip 100b is similar to that in (the modified example of) embodiment 1, that is, as illustrated in Figure 9.

The operation in the present embodiment 3 is similar to that in (the modified example of) embodiment 1 but differs in the following respects. First, since the electrically conductive thin films 130a and 130b are covered with the thin film portion 111b, a near field capable of interaction with a specimen is localized inside the nanopore 120. A remaining near field is confined in the thin film portion 111a and the thin film portion 111b and, therefore, cannot interact with the specimen including a biopolymer. As a result, advantageously, no background signal is produced from a specimen existing in the space other than the interior of the target nanopore 120, and the S/N is high. Second, the near field is formed just at a center of the nanopore 120 in the center axis direction of the same. The motion of a specimen biopolymer such as DNA in the xy direction is therefore limited by the nanopore to enable the specimen to interact with the uniform near field. As a result, advantageously, the reproducibility of the obtained signal is high. Third, DNA is extended along the axial direction in the course of passage through the nanopore 120. Therefore, advantageously, the high-order structure is dissolved, individual bases can be successively introduced into the near field, and the correlation between the sequence observation region of the specimen and measurement times can be simplified, thus facilitating analysis.

### [Embodiment 4] Nanopore chip in which electrically conductive thin film is energized

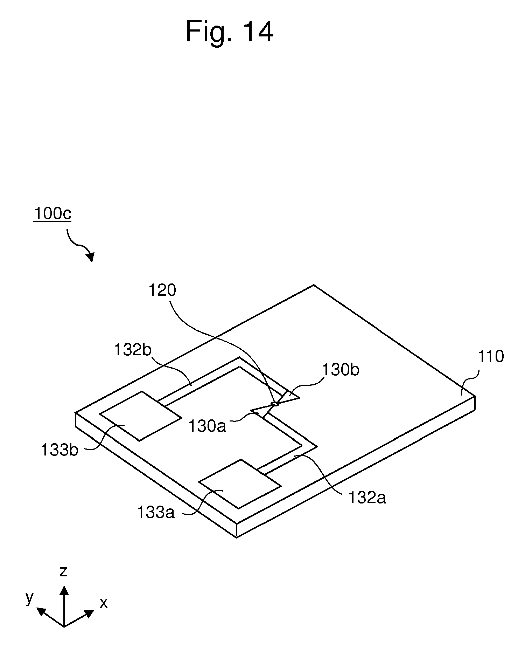

An example of a construction of a nanopore chip for biopolymer characteristic analysis according to the present invention will be described with reference to Figure 14. Figure 14 is a schematic view of a nanopore chip 100c in the present embodiment 4. As illustrated, the nanopore chip 100c includes a substrate 110, a nanopore 120, electrically conductive thin films 130a and 130b, wiring patterns 132a and 132b, and contacts 133a and 133b. Each of the wiring patterns 132a and 132b and the contacts 133a and 133b has electrical continuity to the electrically conductive thin film 130a or 130b.

The wiring patterns 132a and 132b and the contacts 133a and 133b are formed in the same way as the electrically conductive thin films 130a and 130b in the embodiments described above. Points of difference in the forming reside in that gold was used as a material for the wiring patterns and the contacts, and that 1-micron-thick wiring patterns and 100-micron-thick contacts were adopted.

A biopolymer optical analysis apparatus according to the present embodiment 4 is similar to those in embodiments 1 to 3 but differs in the following respect. That is, in the present embodiment 4, opposite-phase connections from the contacts 133a and 133b to a second voltage output of the specimen drive device 700 are made through a card edge connector (not illustrated).

The operation in the present embodiment 4 is similar to those in the above-described embodiments but differs in the following respects. That is, in the present embodiment 4, a voltage in pulse form was applied from the specimen drive device 700 to the specimen chamber to electrophorese a biopolymer through the nanopore 120. A near field was formed at the end portions of the electrically conductive thin films 130a and 130b by laser irradiation. When the pulse from the specimen drive device 700 is OFF, the electrophoresis voltage is cut and an opposite-phase pulse, i.e., an ON pulse, is applied to the contacts 133a and 133b. This pulse voltage is conducted to the electrically conductive thin films 130a and 130b through the wiring patterns 132a and 132b to be applied to the end portions 131a and 131b (not illustrated). A phosphate group in a biopolymer DNA is then attracted to the anode-side end portions of the electrically conductive thin films 130a and 130b. During this state, therefore, electrophoresis of the DNA is temporally and forcibly stopped. During this state, the intensity ratio based on Raman scattered light from the biopolymer is obtained. Similarly, when the pulse from the specimen drive device 700 is ON, the electrophoresis voltage is applied and an opposite-phase pulse, i.e., an OFF pulse, is applied to the contacts 133a and 133b to end the temporary forced stopping of electrophoresis of the DNA, thereby restarting electrophoresis of the DNA. During this state, detection of Raman scattered light is not performed.

In the present embodiment 4, not only pulse drive through the electrophoresis current but also forcible stopping/ending stopping of a biopolymer based on application of a voltage to the electrically conductive thin films is performed in synchronization of the electrophoresis current pulse. As a result, advantageously, the movement of a biopolymer through the nanopore can be controlled with higher accuracy.

### [Embodiment 5] Nanopore chip using graphene as electrically conductive thin film

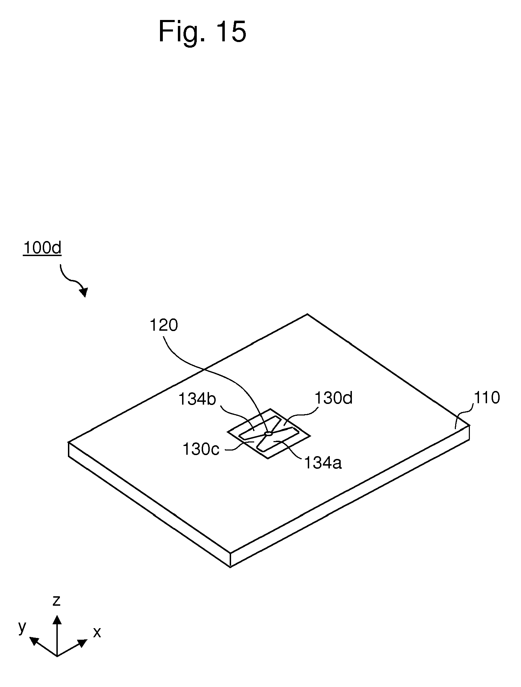

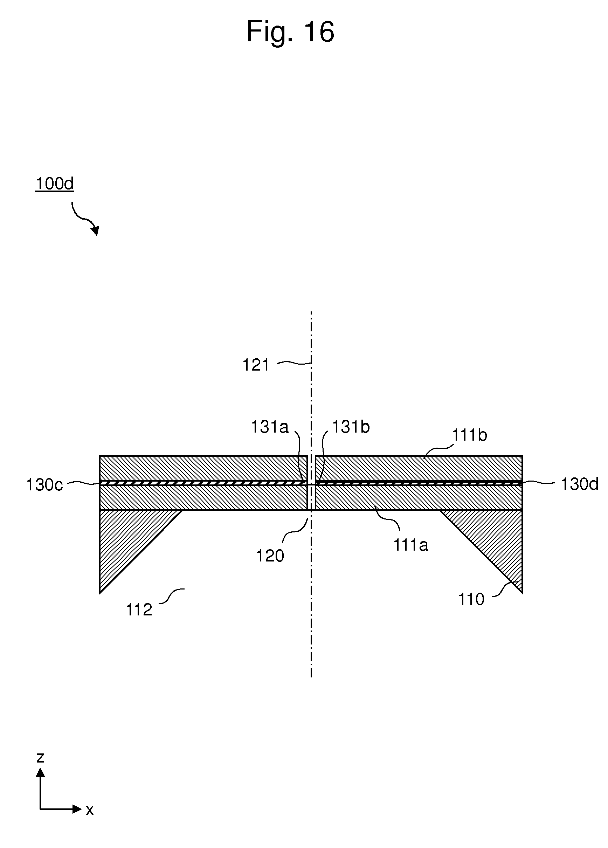

An example of a construction of a nanopore chip for biopolymer characteristic analysis according to the present invention will be described with reference to Figures 15 and 16. Figure 15 is a schematic view of a nanopore chip 100d in the present embodiment 5. The nanopore chip 100d includes a substrate 110, a nanopore 120, and electrically conductive thin films 130c and 130d. As illustrated, the present embodiment 5 is similar to embodiment 3 but differs in the following respects. First, a single layer of graphite, i.e., graphene, was used as electrically conductive thin films 130c and 130d. Second, the planar shapes of the electrically conductive thin films 130c and 130d are connected to each other in surround form in a frame-like structure. In other words, the electrically conductive thin films 130c and 130d are in a structure in the form of one thin film having spaces 134a and 134b. (The spaces 134a and 134b also connect to each other at the nanopore 120). Third, a nanopore having a diameter of 2 nm is adopted as nanopore 120.

Figure 16 is a schematic enlarged view of the nanopore 120 in an xz section containing the center axis 121 of the nanopore 120. The outer frame of the electrically conductive thin films 130c and 130d is not shown in the figure because the enlargement ratio is high.

A method of making the nanopore chip 100d in the present embodiment 5 is similar to that in embodiment 3 but differs in the process after forming of the thin film portion 111a on the substrate 110. That is, graphene was separately made from graphite by using a mechanical exfoliation method and it was confirmed with an optical microscope that graphene was in a single layer. This graphene was transferred onto a supporting substrate for working by using the wedging method described by Schneider et al. in Nano Letters (2010) 10, 1912. By using a high-focus TEM (acceleration voltage 300 kV), an electron beam was applied to the graphene on the supporting substrate to struck out carbon, thereby forming the spaces 134a and 134b and the portion connecting the spaces 134a and 134b. The supporting substrate was taken out of the TEM and the worked graphene was transferred onto the thin film portion 111a of the substrate 110 by using the wedging method again. Thereafter, the thin film portion 111b was formed by sputtering, as in the case of embodiment 3, and the nanopore 120 was formed by using a TEM (by applying an electron beam to the portion connecting the spaces 134a and 134b).

The operation of the present embodiment 5 is similar to that in embodiment 3 but differs in the following respects. First, since the electrically conductive thin films 130c and 130d are formed of graphene, their thickness is extremely small, about 0.3 nm. Accordingly, the thickness of a near field formed between the end portions 131a and 131b is extremely small, 1 nm or less and, advantageously, the spatial resolution is high, about 1 to 3 bases in terms of the number of bases of DNA. That is, advantageously, errors in analysis due to the above-described differential method are reduced and kinds of bases can be identified with higher accuracy. Second, since the electrically conductive thin films 130c and 130d are formed of graphene, the thickness of the end portions 131a and 131b is extremely small and the end portions 131a and 131b are sharply pointed along the thickness direction. Advantageously, if the tips are sharply pointed, the electric field is concentrated so that the effect of enhancing the near field is heightened. Third, since the electrically conductive thin films 130c and 130d are formed of graphene (that is, carbon), they have high stability, for example, against oxidation in an aqueous solution compared to silver. While a single layer of graphene was used in the present embodiment, about 2 to 15 layers of graphene may alternatively be used. Also in a case where such a plurality of layers of graphene or graphite are used, an extremely thin electrically conductive thin film of 5 nm or less can be formed and, therefore, the same effect as that described above can be obtained. Moreover, in such a case, a higher strength can also be obtained as a specific effect. Fourth, since the inside diameter of the nanopore is reduced, the specimen passage speed can be advantageously limited.

As a modified example of the present embodiment 5, a method in which the electrically conductive thin films 130c and 130d are made independent of each other by removing their outer frame and are connected to an external device by leading out wiring in a way such as shown in embodiment 4 can be adopted. Use of a tunnel current measuring device as the external device enables measurement of a tunnel current flowing through a specimen between the end portions 131a and 131b of the electrically conductive thin films 130c and 130d. This modified example is advantageous in that since the thickness of the end portions is small, the spatial resolution in tunnel current measurement is high. A combination of this modified example and one of embodiments 1 to 5 enables simultaneously performing Raman measurement and tunnel current measurement. The reliability of analysis results can be improved by complementarily using the results of the two kinds of measurement. It is also possible to improve the S/N of measurement and improve the reliability of analysis results by detecting the existence of a base of DNA from the results of one of the kinds of measurement and by synchronizing the timing of the other measurement.

The nanopore chip provided with the electrically conductive thin film according to the present invention can also be applied to a fluorescence measurement method, e.g., a nanopore sequencer using a fluorescence probe. In such a case, high sensitivity and high spatial resolution can be achieved by using a near field formed by the electrically conductive thin film.

Further, improving the analysis accuracy by combining two or more of the elements constituting the present invention described above and the prior arts is also effective.

### [Embodiment 6] Two-split-ratio spectroscopic detection device

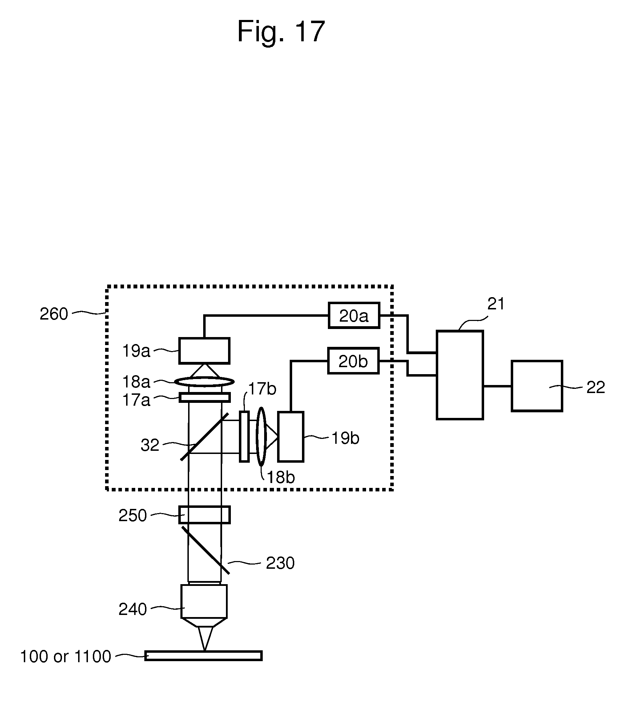

An example of a construction of the detection device in the biopolymer optical analysis apparatus of the present invention will be described with reference to Figure 17. Figure 17 is a construction diagram of the detection device for biopolymer analysis in the present embodiment 6. Components corresponding to the light source and the beam expander shown in Figures 3 and 12 are omitted.

The nanopore chip 100 or the multi-nanopore chip 1100 is used as a measurement substrate. A case where the multi-nanopore chip 1100 is used will be described below. Raman scattered lights derived from a specimen are independently produced from a plurality of nanopores 120 on the substrate. The Raman scattered lights are condensed by the objective lens 240 and pass the dichroic mirror 230. After Rayleigh scattered light has been removed by the filter 250, the Raman scattered lights are detected by the two-split-ratio spectroscopic detection device 260. the two-split-ratio spectroscopic detection device 260 includes a dichroic mirror for two-split-ratio spectroscopic detection 32, auxiliary filters 17a and 17b, image-forming lenses 18a and 18b, two-dimensional sensor cameras 19a and 19b, and two-dimensional sensor camera controllers 20a and 20b.

The condensed Raman scattered lights are separated into beams split at different ratios for each wavelength by the dichroic mirror for two-split-ratio spectroscopic detection 32. The separated beams pass through auxiliary filters 17a and 17b and form images on two-dimensional sensor cameras 19a and 19b (high-sensitivity cooled two-dimensional CCD cameras) through the image-forming lenses 18a and 18b, and the images are detected. Through the two-dimensional sensor camera controllers 20a and 20b, a control PC 21 controls operations including setting of the camera exposure time and timing of taking in of optical images. As the filter unit 250, a combination of a notch filter for removing laser light of the light source and a band-pass interference filter through which light in a wavelength band to be detected can pass is used. As the notch filter for removing laser light, a filter having a well-known characteristic and designed specially for the laser to be used is used. Figure 18(A) schematically shows characteristics of the band-pass interference filter (and the auxiliary filters 17a and 17b), and Figure 18(B) schematically shows a characteristic of the dichroic mirror for two-split-ratio spectroscopic detection 32. The abscissa represents the wave number.

The apparatus includes an automatic focusing mechanism, devices for substrate position adjustment or the like, i.e., a transmitted light illumination, a reflected light illumination, a transmitted light/reflected light observation lens barrel and a TV camera (not illustrated), and a monitor 22. The state of the substrate 1100 can be observed in real time with a halogen illumination or the like.

As the two-dimensional sensor cameras 19a and 19b used in the present embodiment, a CCD area sensor is used. Any of CCD area sensors of various pixel sizes and numbers of pixels can be used. For example, a high-sensitivity electron-multiplying cooled CCD camera having a pixel size of 16 x 16 micrometers and a 512 x 512 number of pixels is used. As the two-dimensional sensor cameras, an image-taking camera such as a C-MOS area sensor can ordinarily be used as well as the CCD area sensor. The CCD area sensor also includes a back-illuminated type and a front-illuminated type based on different structures. Either of these types is usable. An electron multiplier CCD camera (EM-CCD) incorporating a signal multiplying function in the device, as in the present embodiment, is also effective in improving the sensitivity. Preferably, the sensor is of a cooled type. If the sensor is maintained at a temperature equal to or lower than about -20°C, dark noise that the sensor has can be reduced and the accuracy of measurement can be improved.

Necessary pixels of the CCD per nanopore are about 3 x 3 pixels or more. A bright spot of Raman scattering in the nanopore is detected at a center of the pixels. A 512 x 512 pixel CCD area sensor is capable of simultaneously detecting and discriminating Raman scattering at 29000 nanopores at the maximum. If the distance at which nanopores are spaced apart from each other is increased, the number of nanopores on which measurements can be simultaneously made is reduced. If the size of nanopores is larger than that, or if the substrate size is larger, measurements can be separately made by sectioning the area. In such a case, an X-Y movement mechanism unit to move the substrate is disposed below the stage, and the control PC 21 controls the movement to the irradiation position, application of light and detection of optical images. The X-Y movement mechanism unit is not illustrated in the present embodiment.

Detection and discrimination of Raman scattered light will next be described. To be specific, a system will be described in which Raman scattered light derived from a specimen is detected from each of a plurality of nanopores of the multi-nanopore chip 1100, the corresponding specimen component, i.e., the kind of a base is identified and the intensity is measured.

Referring to Figure 17, the total image-forming magnification on the two-dimensional sensor cameras (CCD cameras) 19a and 19b is set to 20, and Raman scattered light from each nanopore is measured. In a case where the nanopores in the multi-nanopore chip 1100 are disposed in a 4 x 4 micrometer lattice array, the interval between each pair of the nanopores is divided into five to be detected through CCD pixels.

For example, in a case where wavelength dispersion is performed by using a dispersion element, the smallest interval between the lattice points is 4 micrometers and corresponds to five pixels in terms of number of CCD pixels. Dispersion of a Raman scattering spectrum within a five-pixel range is performed, and it is virtually difficult to perform spectral separation. Further, in an actually formed image, the emission bright spot at each nanopore spreads without being formed within one pixel and is, therefore, formed over several pixels, and overlaps are increased, so that spectral separation becomes more difficult.

In the present embodiment, such a restriction is removed since Raman scattered light from each nanopore forms an image without being dispersed. The present embodiment is effective in measurement on a chip on which multiple nanopores are disposed at a high density.

Raman scattered lights from bases (for example, Figure 7) reach the two-dimensional CCD cameras 19a and 19b at different ratios. At the two-dimensional CCD cameras 19a and 19b, no information is obtained along the wavelength direction; only brightness information on bright spots is obtained. More specifically, the Raman scattered lights are separated into reflected light images and transmitted light images determined by the products of the spectra shown in Figure 7 and the spectrum shown in Figure 18(B), and corresponding bright spots are obtained. Figure 8 shows spectral distributions and the ratios in the bright spots when the bright spots are Raman scattering images respectively derived from A, C, G, and T. In Figure 8, (A) shows transmitted light spectrum components from the bases corresponding to the Raman scattered light shown in Figure 7, and (B) shows reflected light spectrum components. Since the Raman scattering spectra from the bases (Figure 7) differ from each other, the spectrum components transmitted through the dichroic mirror for two-split-ratio spectroscopic detection 32 and the spectrum components reflected by this mirror vary for each kind of base, and the detected transmission intensity and reflection intensity vary correspondingly. That is, as shown in (C) of Figure 8, the ratio of the transmission intensity to the total intensity varies for each kind of base, and the kinds of bases can be identified from this ratio, thus enabling determination of bases passing through the nanopore. Thus, discrimination and detection of Raman scatterings from the four kinds of bases can be performed by using the dichroic mirror that splits light at different ratios for each wavelength, splitting the intensity of light from one bright spot, and detecting the split intensities through different pixels. The control PC 21 extracts the bright spots at the nanopores from the images on the CCD cameras, calculates the ratios of the intensities, and identifies the kinds of bases.

The characteristic of the usable dichroic mirror is not limited to that shown in the present embodiment. A dichroic mirror of a different characteristic is applicable if four or more types of Raman scattering having combinations of intensities in which the reflection intensity and the transmission intensity vary (are discriminable) can be obtained from the characteristic. The arrangement can be applied to an RNA sequence, etc., as well as to the base sequence of DNA.

The provision of the two two-dimensional CCD cameras 19a and 19b is not necessarily required. One two-dimensional CCD camera may suffice. When, in such a case, split images are formed on one two-dimensional CCD camera, their positions may be relatively changed, for example, by being laterally shifted.

While the present embodiment has been described with respect to identification of four kinds of bases, identification of three, five, six or more kinds of bases can be performed.

In the present embodiment, a dichroic mirror having a generally linear transmittance characteristic from about 0% to about 100% in a prescribed wave number range is used as dichroic mirror 32. However, a transmittance characteristic generally linear from about 10% to about 80% may suffice. A split mirror having such a characteristic as to have different transmittances and reflectances for each of arbitrary wave number bands according to a plurality of scattering spectra to be used may alternatively be used. For example, a mirror (schematically shown in Figure 19) having a characteristic that changes in steps with wave numbers corresponding to characteristic Raman scattering peaks is also applicable.

In the present embodiment, a plurality of nanopores are precisely disposed; Raman scattered lights derived from a biopolymer entering the nanopores are caused to form images on particular pixels of a plurality of detectors having a plurality of detection pixels; and the intensity ratios are measured on a detector-by-detector basis, thus enabling identification of the kinds of monomers constituting a biopolymer. In particular, three or more kinds of monomers can be identified and detected with one or two two-dimensional CCD cameras provided as detectors. The apparatus cost can be limited in this way. Needless to say, detection can be performed on a molecule-by-molecule basis.

Also, in the present embodiment, the substrate having nanopores disposed at certain intervals in a lattice array is used. The disposition of nanopores in a lattice array facilitates discrimination of bright spots in the transmission image and bright spots in the reflection image corresponding to each other. A modification can be made for a case where the substrate having capture regions disposed in a lattice array is not used. Even in a case where capture regions are randomly disposed, comparison between the two images and pattern analysis or determination of corresponding bright spots by referring to a reference marker may be performed. The same effect can also be obtained in this way.

The number of CCD pixels per emission bright spot has been assumed to be 5 x 5 pixels. However, an adjustment, for example, to 4 x 4 pixels, 5 x 4 pixels, 4 x 3 pixels, or 3 x 3 pixels may alternatively be made. Also in the case of detection with any of these numbers of pixels, high-efficiency detection and identification of molecules captured at a high density can be performed.

### [Embodiment 7] Another construction of two-split-ratio spectroscopic detection device

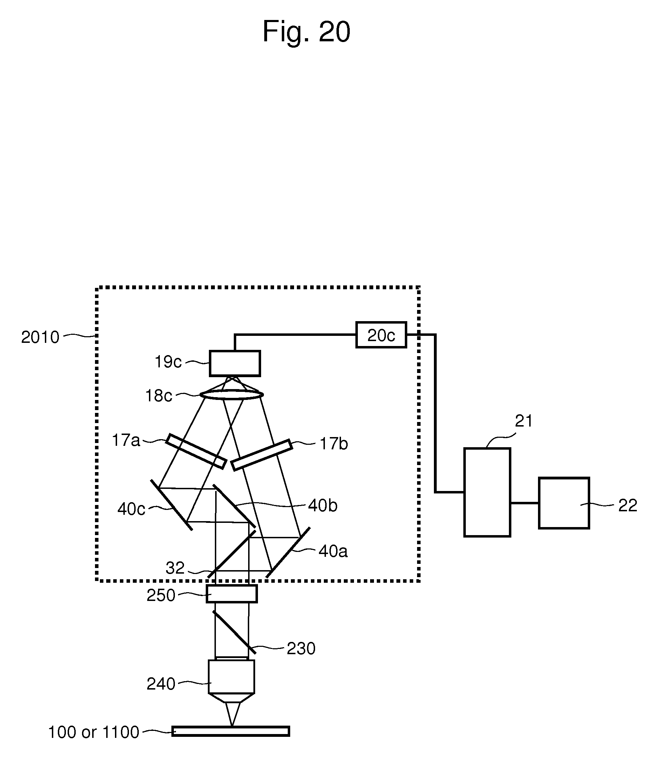

An example of another construction of the detection device in the biopolymer optical analysis apparatus of the present invention will be described. Figure 20 is a construction diagram of a detection device for biopolymer characteristic analysis in the present embodiment 7. Components corresponding to the light source and the beam expander shown in Figures 3 and 12 are omitted, as in the case of Figure 17.

The nanopore chip 100 or the multi-nanopore chip 1100 is used as a measurement substrate. A case where the multi-nanopore chip 1100 is used will be described below. Raman scattered lights derived from a specimen are independently produced from a plurality of nanopores on the substrate. The Raman scattered lights are condensed by the objective lens 240 and pass the dichroic mirror 230. After Rayleigh scattered light has been removed by the filter 250, the Raman scattered lights are detected by the two-split-ratio spectroscopic detection device 2010. The two-split-ratio spectroscopic detection device 2010 includes a dichroic mirror for two-split-ratio spectroscopic detection 32, mirrors 40a, 40b, and 40c, auxiliary filters 17a and 17b, an image-forming lens 18c, a two-dimensional sensor camera 19c, and a two-dimensional sensor camera controller 20c.

First, splitting into beams of transmitted light and reflected light at different ratios for each wavelength with the splitting dichroic mirror 32 is performed. The split beams of reflected light are respectively reflected by the mirror 40a and led to the image-forming lens 18c at a prescribed angle. The beams of transmitted light are bent by the mirrors 40b and 40c and led to the same image-forming lens 18c at another prescribed angle. The two beams enter the image-forming lens 18c at different angles to respectively form images in a state of being relatively shifted on the two-dimensional sensor camera 19c (high-sensitivity cooled two-dimensional CCD camera). As auxiliary filters 17a and 17b, band-pass interference filters are used for removal of stray light. Use of the auxiliary filters is not required in some case. The images on the two-dimensional sensor camera 19c are collected and analyzed by the control PC 21 through the two-dimensional sensor camera controller 20c. The lengths of the optical paths for the split transmitted light and the split reflected light are set as close to each other as possible. However, the optical path lengths are not necessarily set equal to each other. While in the illustration the image-forming magnifications for the two beams are equal to each other, they may set different from each other. The images can be analyzed as long as the split transmitted light image and the split reflected light image for the same bright spot can be identified.

In the present embodiment, Raman scattered light derived from a plurality of nanopores can be detected with one two-dimensional CCD camera and, therefore, the measurement process can be simplified and there is no need for sensitivity correction due to an individual difference of the two-dimensional CCD camera, and for control of measurement timing. As a result, the measurement accuracy is improved.

### [Embodiment 8] Characteristic analysis chip having channel with projection structure

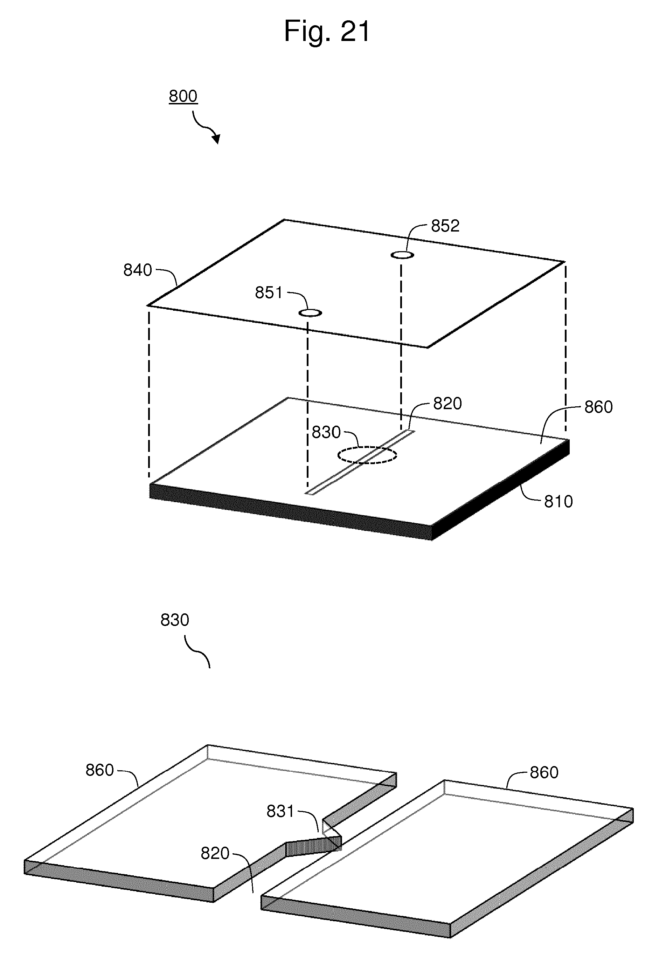

One embodiment of a construction of a nano-channel chip for biopolymer characteristic analysis in the present embodiment will be described with reference to Figure 21. A nano-channel chip 800 includes a substrate 810, a channel 820 and a cover glass 840. An opening portion 851 for introducing a specimen to be measured and an opening portion 852 for leading out the specimen are provided on the cover glass 840 at positions corresponding to opposite ends of the channel 820. The channel 820 is formed by an electrically conductive thin film on the substrate 810. In a central observation region 830, a projection structure 831 is provided and the enhancement field described in embodiment 1 is produced. That is, a projecting shape (projection structure) having a pointed end in one place in the channel is formed. The projecting shape is formed on one of opposite wall surfaces along the flow direction of the channel.

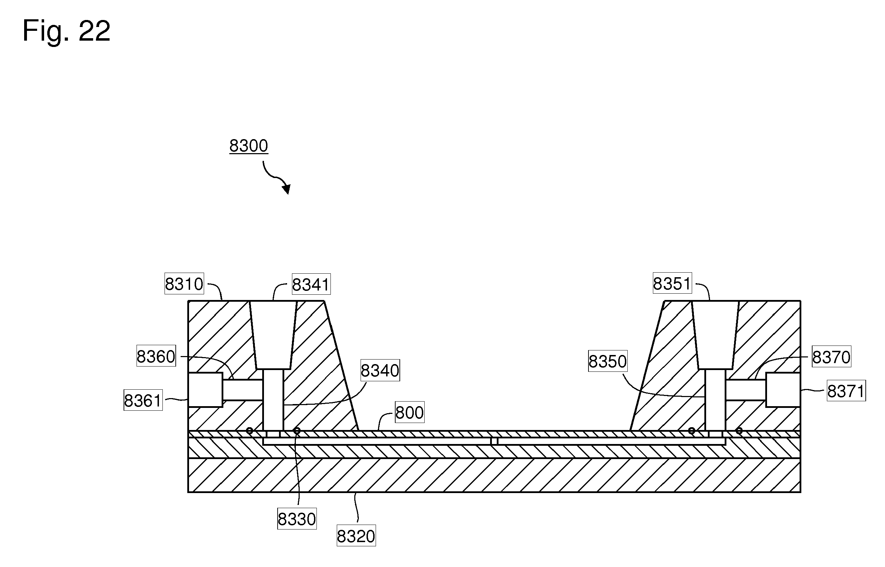

The construction of an analysis apparatus for performing biopolymer characteristic analysis in the present embodiment is similar to that in embodiment 1 (see Figure 3) but differs in the construction of the specimen cell. Figure 22 is a sectional view schematically showing the construction of a specimen cell 8300. The specimen cell 8300 includes a nano-channel chip 800, an upper member 8310, a lower member 8320, and screws (not illustrated). In the upper member 8310, an O-ring 8330, specimen channels 8340 and 8350, electrode channels 8360 and 8370, specimen connection ports 8341 and 8351, electrode connection ports 8361 and 8371 are formed. Specimen feed tubes (not illustrated) are connected to the upper member 8310 through the specimen connection ports 8341 and 8351 in an airtight manner. Silver-silver chloride electrodes (not illustrated) are also connected to the upper member 8310 through the electrode connection ports 8361 and 8371 in an airtight manner. Each of the silver-silver chloride electrodes has silver chloride on one end, and this end is housed in an electrode channel chamber (not illustrated). The silver-silver chloride electrode has silver on its other end (hereinafter referred to as "silver end"), and this end is exposed outside the electrode channel. The above-described specimen feed tubes, specimen channels 8340 and 8350, specimen connection ports 8341 and 8351, electrode channels 8360 and 8370 and electrode connection ports 8361 and 8371 are filled with a specimen solution (not illustrated) leaving no space therein (without mixing in air bubbles). As a result, the specimen solution in the specimen cell 8300 and the nano-channel chip 800 contacts the silver-silver chloride electrodes, and electrochemical continuity is established therebetween.

The outline of the operation in the present embodiment will be described with reference to Figures 3, 21, and 22. The specimen cell 8300 is first prepared. To be specific, the nano-channel chip 800 is interposed between the upper member 8310 and the lower member 8320 and maintained in pressure contact with O-rings 8330, thereby connecting the specimen channels 8340 and 8350 and the opening portions 851 and 852 of the nano-channel chip 800 in an airtight manner. A 100 mM KCl aqueous solution is introduced as a specimen solution from the specimen feed tube to fill the specimen channels 8340 and 8350 of the specimen cell 8300, the electrode channels 8360 and 8370, the specimen connection ports 8341 and 8351, the electrode connection ports 8361 and 8371 and the channel 820 in the nano-channel chip 800 with the specimen solution.

Next, the specimen cell 8300 is set in the analysis apparatus 200. More specifically, the specimen cell 8300 is fixed on the slightly-movable stage 600. The optical system is focused on observation region portion 830 of the nano-channel chip 800 in the specimen cell 8300 by using the slightly-movable stage 600 and an optical system for visual inspection not illustrated. The two silver-silver chloride electrodes set on the specimen cell 8300 are connected to the specimen drive device 700. The specimen drive device 700 incorporates a voltage source or a current source and can apply a voltage to the electrode connection port 8371 on the specimen lead-out side with respect to the electrode connection port 8361 on the specimen introduction side.

The optical system of the analysis apparatus and a procedure of measurement of DNA to be measured are the same as those described in embodiment 1. Changes with time in enhanced Raman scattered light when a specimen to be measured passes by electrophoresis through a near field formed at the end portion 831 of the electrically conductive thin film 860 are obtained.

A method of making the nano-channel chip in the present embodiment is the same as the method of making the electrically conductive thin film 130 in (the modified example of) embodiment 1. Gold thin film 860 was formed on the silicon substrate 810; a resist was applied; a pattern for the measurement region 830 shown in Figure 21 was formed by EB lithography; gold in the other region was removed by etching; the resist was removed; and a cover glass 840 was finally put on and fixed. The size of the projection structure 831 is equal to that in embodiment 1. The width of the channel 820 interposed between the projection structure 831 and the electrically conductive thin film 860 is about 30 nm. This is substantially equal to the opening diameter of the nanopore described in embodiment 1, which allows a DNA molecule for example to pass without clumping.

While gold was used as the material of the electrically conductive thin film 860 in the present embodiment, this material is not limited to gold. Any of general materials may be used if it has electrical conductivity. In ordinary cases, a metal can be preferably used. Examples of other metals usable as electrically conductive thin film are platinum group metals, such as platinum, palladium, rhodium and ruthenium, silver and copper.

The material of the cover glass 840 used in the present embodiment is, for example, glass, quartz and a plastic material such as acrylic having high transparency at the light source wavelength.

In the present embodiment, a specimen to be measured moves parallel to the substrate 810 along the channel. That is, the biopolymer characteristic analysis chip in the present embodiment differs from that in embodiment 1 in having a nanosize channel instead of a nanopore. The advantage of this structure resides in that it is not necessary to provide specimen chambers on both the upper and lower sides of the substrate. In embodiment 1, since the specimen chamber exists between the objective lens and the chip, there is a possibility of background light being increased by a signal produced from the specimen solution in the chamber in observation. In the present embodiment, the influence of this can be eliminated, so that noise components are reduced. Further, because of the removal of the specimen chamber, the objective lens can be brought closer to the measurement region than in any other known cases. Accordingly, use of a lens having a higher NA compared with that in embodiment 1 is enabled and the signal intensity can be improved. As a result, observation at a higher S/N compared with embodiment 1 can be performed.

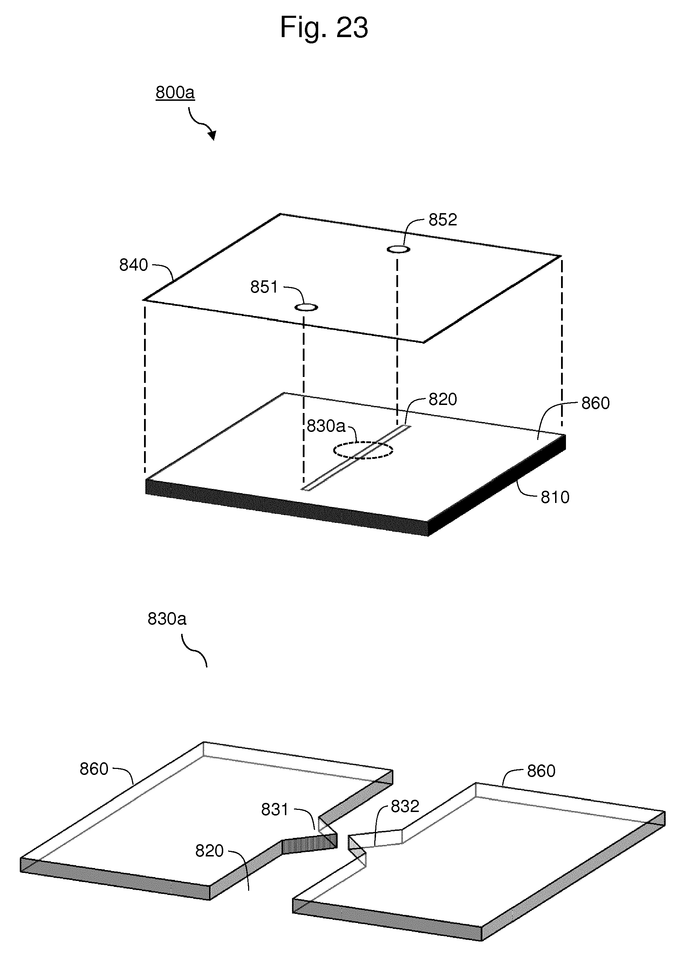

As a modified example close to the present embodiment, a nano-channel chip 800a of a construction described below can be implemented. Figure 23 is a schematic view of a nano-channel chip 800a in the present modified example. The basic construction is similar to the above-described embodiment and Figure 21 but differs from Figure 21 (having only one projecting shape 831) in that the nano-channel chip 800a has two projecting shapes 831 and 832 in a measurement region, as illustrated. End portions of the two projecting shapes are opposed to each other from the left and right wall surfaces of the channel. The distance between the end portions is about 30 nm. This distance is substantially equal to the opening diameter of the nanopore as in the case shown in Figure 21, which allows a DNA molecule for example to pass without cohering.

An analysis apparatus and operation for the nanopore chip 800a of the present modified example are also the same. Features of the effects of the present modified example is also the same as that described above. Also, a plurality of the channels 820 described in the present embodiment or the modified example can be two-dimensionally disposed on the substrate 810 surface.

### [Embodiment 9] Characteristic analysis chip having channel with one row of nanoparticles

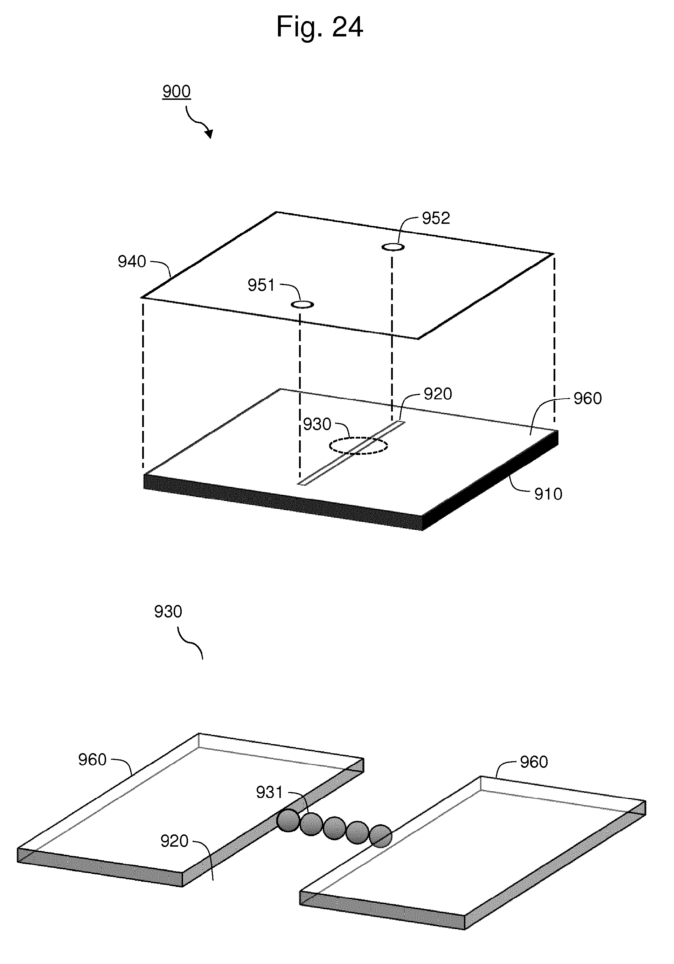

One embodiment of a construction of a nano-channel chip for biopolymer characteristic analysis in the present embodiment will be described with reference to Figure 24. A nano-channel chip 900 includes a substrate 910, a channel 920 and a cover glass 940. An opening portion 951 for introducing a specimen to be measured and an opening portion 952 for leading out the specimen are provided on the cover glass 940 at positions corresponding to opposite ends of the channel 920. The channel 920 is formed by an electrically conductive thin film 960 on the substrate 910. In a central observation region 930, nanoparticles 931 are fixed in a row through the entire width of the channel 920 to produce the enhancement field described in embodiment 1. As illustrated, the present embodiment is similar to embodiment 8 but differs in that no projecting shapes exist in the measurement region and nanoparticles are fixed instead. In other respects, the present embodiment is the same as embodiment 8.

A method of making the chip in the present embodiment is as described below. First, by the procedure in embodiment 8, the nanosize channel 920 is made. A resist is applied on the substrate surface, and a channel pattern is formed by EB lithography. Next, gold nanoparticles 931 are fixed. A resist is applied on the entire substrate 910 including the channel 920, and a pattern for fixing the gold nanoparticles 931 on the channel is formed by EB lithography (positive). Post baking is performed, followed by rinsing with pure water, thereby achieving a state where part of the channel is exposed. SiO₂ thin film is formed on the exposed portion by sputtering. The resist is thereafter removed by acetone. A state where a thin film of SiO₂ exists on a portion of the bottom of the channel 920 results. By reaction with a silane coupling agent, a functional group is added on the SiO₂ thin film. The gold nanoparticles 931 are caused to react with the functional group and bond to the same. Finally, the cover glass 940 is put on and fixed.

The operation in the present embodiment 9 is the same as that in (the modified example of) embodiment 1. In the case where the gold nanoparticles 931 are used, an enhancement field is produced on the periphery of each particle and in the gaps between the particles. The gold nanoparticles 931 are most closely packed in the channel 920 by setting the height of the channel 920 (equal to the film thickness of the electrically conductive thin film 960) and the diameter of the gold nanoparticles 931 equal to each other and by setting the width of the channel 920 to an integer multiple of the diameter of the gold nanoparticles 931. As a result, a specimen to be measured can pass through the enhancement field.

In the present embodiment, the same effects as those in embodiment 8 can be obtained by using the gold nanoparticles 931. Also, a characteristic effect of enabling the chip to be made comparatively easily in comparison with embodiment 8 is obtained.

While gold was used as the material of the nanoparticles 931 in the present embodiment, this material is not limited to gold. Any of general materials may be used if it has electrical conductivity. In ordinary cases, a metal can be preferably used. Examples of other metals usable as electrically conductive thin film are platinum group metals, such as platinum, palladium, rhodium and ruthenium, silver and copper. The same effect can also be obtained by fixing resin beads, thereafter surface-coating any of various metals by sputtering and removing the resin components by heating for example.

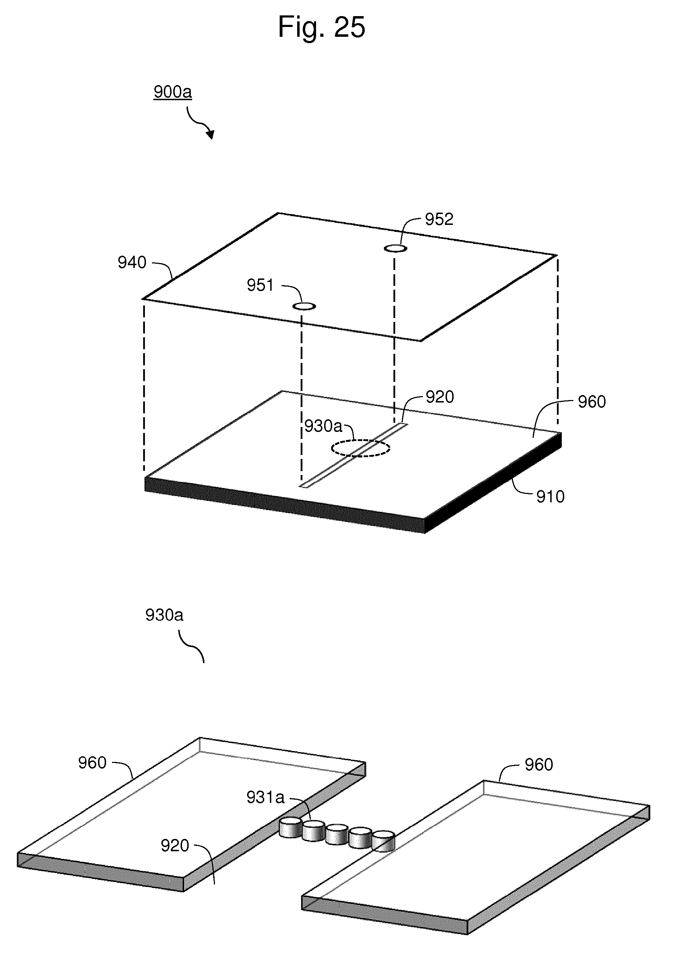

As a modified example close to the present embodiment, a nano-channel chip 900a of a construction described below can be implemented. Figure 25 is a schematic view of a nano-channel chip 900a in the present modified example. The basic construction is similar to Figure 24 but differs from the case of Figure 24 in that the nano-channel chip 900a has cylindrical metal structural members 931a in a measurement region 930a, as illustrated.

A making method according to the present modified example is based on EB lithography, as is the method of making the channel 920. An analysis apparatus and operation for the nano-channel chip 900a of the present modified example are the same as those described in embodiment 8. Features of the effects of the present modified example are the same as those described in embodiment 8. While the cylindrical metal structural members 931a are disposed in the present modified example, each member may alternatively be formed into the shape of a polygonal prism such as a triangular prism or a rectangular prism. Also, a plurality of the channels 920 described in the present embodiment or the modified example can be two-dimensionally disposed on the substrate 910 surface.

### [Embodiment 10] Characteristic analysis chip having channel with a plurality of rows of nanoparticles

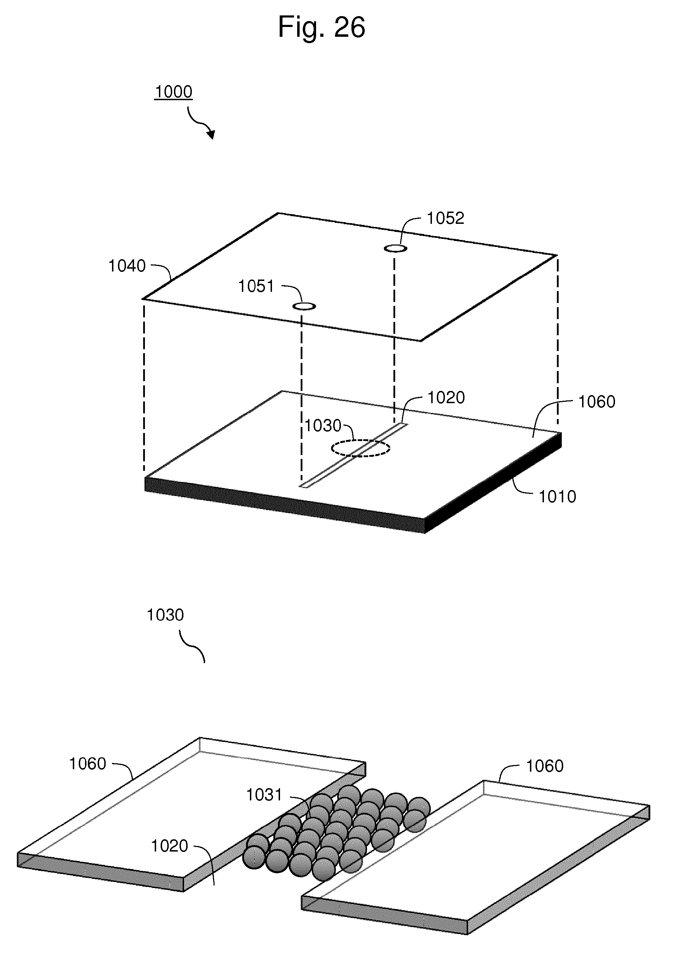

One embodiment of a construction of a nano-channel chip for biopolymer characteristic analysis in the present embodiment will be described with reference to Figure 26. A nano-channel chip 1000 includes a substrate 1010, a channel 1020 and a cover glass 1040. An opening portion 1051 for introducing a specimen to be measured and an opening portion 1052 for leading out the specimen are provided on the cover glass 1040 at positions corresponding to opposite ends of the channel 1020. The channel 1020 is formed by an electrically conductive thin film 1060 on the substrate 1010. In a central observation region 1030, nanoparticles 1031 are fixed in a plurality of rows through the entire width of the channel 1020 to produce the enhancement field described in embodiment 1. As illustrated, the present embodiment is similar to embodiment 9 but differs in that a plurality of rows of nanoparticles 1031 are fixed instead of one row. In other respects, the present embodiment is the same as embodiment 9.

A chip making method in the present embodiment is the same as that in embodiment 9. The operation in the present embodiment is the same as (the modified example of) embodiment 8 but differs in the following respects. In the case where gold nanoparticles are used, an enhancement field is produced on the periphery of each particle and in the gaps between the particles. Therefore, an enhancement field can be produced in a wider area in comparison with embodiment 8. While the enhancement field in embodiment 8 is limited to an area having a diameter of 30 nm on the tip end portion of the projecting shape, a feature of the present embodiment resides in that the area of the enhancement field can be increased to the size of the laser light irradiation spot at the maximum. Another feature of the present embodiment resides in that the signal intensity per pixel can be increased in comparison with embodiments 8 and 9.

When observed at an enlargement magnification of 20 with an EM-CCD having a pixel size of 16 µm/pixel, one pixel of the CCD corresponds to 0.8 µm on the nano-channel chip 1000. In a case where the diameter of the nanoparticles 1031 is 0.1 µm and the nanoparticles 1031 are fixed in a most closely packed state in the channel 1020, about 10 rows of gold nanoparticles 1031 exist along the flow direction of the channel 1020 in the region corresponding to one CCD pixel. That is, the signal intensity exhibited by one pixel is the sum of enhancement effects of gold nanoparticles 1031 in the ten rows. For example, if the time during which a specimen to be measured exists in the enhancement field when the electrophoresis speed is 1 in embodiment 9 is assumed to be 1, the time during which a specimen to be measured exists in the enhancement field when the electrophoresis speed is 1 in the present embodiment is 10. That is, use of the nano-channel chip 1000 having the above-described construction enables obtaining the same effects as those in embodiment 9 when the electrophoresis speed is about 10 times higher.

In the present embodiment, as described above, enhancement observation with higher efficiency in a simpler way is enabled. The present embodiment is particularly suitable for measurement on a biomonomer carrier.

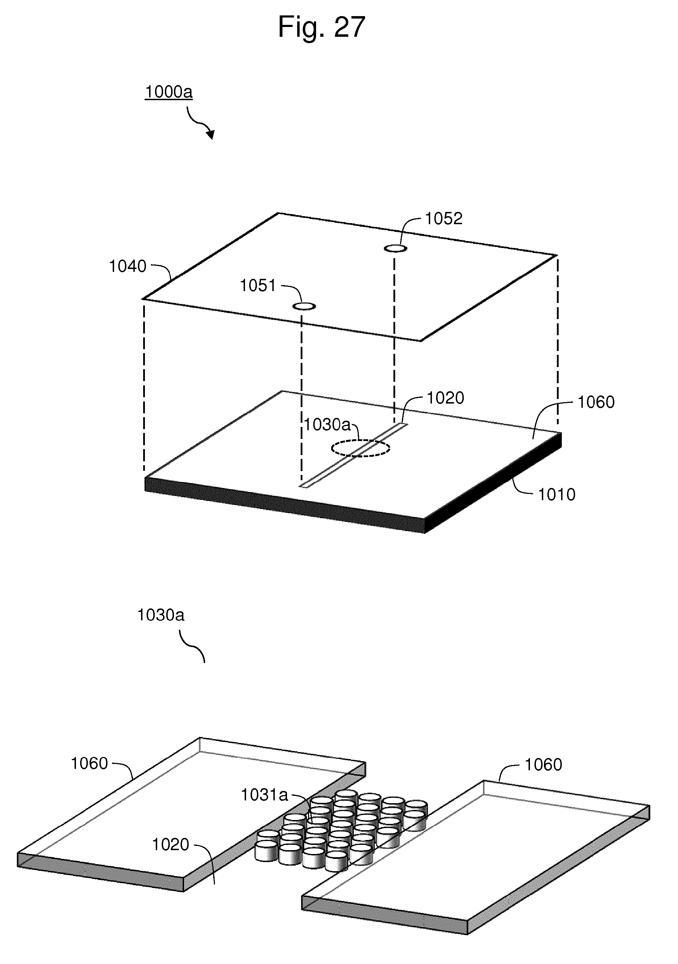

As a modified example close to the present embodiment, a nano-channel chip 1000a of a construction described below can be implemented. Figure 27 is a schematic view of a nano-channel chip 1000a in the present modified example. The basic construction is similar to Figure 26 but differs from the case of Figure 26 in that the nano-channel chip 1000a has cylindrical metal structural members 1031a in a measurement region 1030a, as illustrated.

A making method according to the present modified example is based on EB lithography, as is the method of making the channel 1020. An analysis apparatus and operation for the nano-channel chip 1000a of the present modified example are the same as those described in embodiment 8. Features of the effects of the present modified example are the same as those described in embodiment 8. While the cylindrical metal structural members 1031a are disposed in the present modified example, each member may alternatively be formed into the shape of a polygonal prism such as a triangular prism or a rectangular prism. Also, a plurality of the channels 1020 described in the present embodiment or the modified example can be two-dimensionally disposed on the substrate 1010 surface.

The entire contents of all the publications, patents and patent applications cited in this specification are incorporated in this specification by reference.

### Industrial Applicability

The present invention provides an apparatus and a method for optical analysis of a biopolymer. The analysis apparatus of the present invention is based on a solid-state nanopore method and therefore has high structural stability and high reliability and is capable of two-dimensionally analyzing characteristics of a biopolymer with high spatial resolution and high sensitivity through Raman scattering. The analysis apparatus and the analysis method of the present invention are capable of analyzing a plurality of biopolymers in a plurality of nanopores simultaneously and are, therefore, simple and convenient and have a high throughput. Therefore, the present invention is useful in a field where there is a demand for analysis of characteristics of biopolymers, e.g., in the fields of biotechnology, biochemistry, medical services, and the like.

### Reference Signs List

- 17a, 17b: Auxiliary filter
- 18a, 18b, 18c: Image-forming lens
- 19a, 19b, 19c: Two-dimensional sensor camera
- 20a, 20b, 20c: Two-dimensional sensor camera controller
- 21: Control PC
- 22: Monitor
- 32: Dichroic mirror for two-split-ratio spectroscopic detection
- 40a, 40b, 40c: Mirror
- 100, 100a, 100b, 100c, 100d: Nanopore chip
- 110: Substrate
- 111, 111a: Thin film portion of substrate
- 111b: Thin film portion
- 112: Window portion of substrate
- 120: Nanopore
- 121: Center axis of nanopore
- 130, 130a, 130b, 130c, 130d: Electrically conductive thin film
- 131, 131a, 131b: End portion of electrically conductive thin film
- 132a, 132b: Wiring pattern
- 133a, 133b: Contact
- 134a, 134b: Space
- 200: Analysis apparatus
- 210: Light source
- 220: Beam expander
- 230: Dichroic mirror
- 240: Objective lens
- 250: Filter, filter unit
- 260: Two-split-ratio spectroscopic detection device
- 270: Terminal end
- 300: Specimen cell
- 310: Upper member
- 320: Lower member
- 330: O-ring
- 410, 420, 430: Specimen channel
- 440: (Lower) Specimen chamber
- 450: Electrode chamber
- 460, 470: Specimen connection port
- 480: (Lower) Electrode connection port
- 540: (Upper) Specimen chamber
- 580: (Upper) Electrode connection port
- 600: xyz slightly-movable stage
- 700: Specimen drive device
- 800, 800a: Nano-channel chip
- 810: Substrate
- 820: Channel
- 840: Cover glass
- 851, 852: Opening portion
- 830, 830a: Observation region, measurement region
- 831, 832: Projection structure, end portion
- 860: Electrically conductive thin film
- 900, 900a: Nano-channel chip
- 910: Substrate
- 920: Channel
- 930, 930a: Observation region, measurement region
- 931: Nanoparticle
- 931a: Cylindrical metal structural member
- 940: Cover glass
- 951, 952: Opening portion
- 960: Electrically conductive thin film
- 1000, 1000a: Nano-channel chip
- 1010: Substrate
- 1020: Channel
- 1030, 1030a: Observation region, measurement region
- 1031: Nanoparticle
- 1031a: Cylindrical metal structural member
- 1040: Cover glass
- 1051, 1052: Opening portion
- 1060: Electrically conductive thin film
- 1100: Multi-nanopore chip
- 1110: Substrate
- 1120, 1121: Nanopore
- 1130a, 1130b, 1131a, 1131b: Electrically conductive thin film
- 2000: Multi-analysis apparatus
- 2010: Two-split-ratio spectroscopic detection device
- 8300: Specimen cell
- 8310: Upper member
- 8320: Lower member
- 8330: O-ring
- 8340: Specimen channel
- 8341: Specimen connection port
- 8350: Specimen channel
- 8351: Specimen connection port
- 8360: Electrode channel
- 8361: Electrode connection port
- 8370: Electrode channel
- 8371: Electrode connection port

## Claims

1. A biopolymer optical analysis apparatus comprising:
a biopolymer characteristic analysis chip comprising a solid substrate, a plurality of nanopores provided in the solid substrate, and an electrically conductive thin film disposed on the solid substrate, wherein at least a portion of the electrically conductive thin film faces the nanopores;
a light source and an irradiation optical system for producing Raman scattered lights from a biopolymer entering the nanopores; and
a detection device comprising a Raman scattered light collecting system, a separating component that splits collected light into transmitted light and reflected light at different ratios in a particular wavelength range, an image-forming optical system through which the split lights form images on a detector, and a two-dimensional detector for detecting the lights forming the images,
wherein external light is applied from the light source and the irradiation optical system to the characteristic analysis chip, and Raman scattered lights from the biopolymer in the analysis chip are detected in the detection device.

2. A biopolymer optical analysis apparatus comprising:
a biopolymer characteristic analysis chip comprising a solid substrate, a plurality of nanopores provided in the solid substrate, and an electrically conductive thin film disposed on the solid substrate, wherein at least a portion of the electrically conductive thin film faces the nanopores;
a light source and an irradiation optical system for producing Raman scattered lights from a biopolymer entering the nanopores; and
a detection device comprising a Raman scattered light collecting system, a separating component that splits collected light into transmitted light and reflected light at different ratios for each of prescribed wavelength bands, an image-forming optical system through which the split lights form images on a detector, and a two-dimensional detector for detecting the lights forming the images,
wherein external light is applied from the light source and the irradiation optical system to the characteristic analysis chip, and Raman scattered lights from the biopolymer in the analysis chip are detected in the detection device.

3. The biopolymer optical analysis apparatus according to Claim 1, wherein the biopolymer is constituted by at least four kinds of monomers, and the separating component splits the light at different ratios such that at least four intensity ratios between the transmitted light and the reflected light are discriminated for each of the kinds of monomers constituting the biopolymer.

4. The biopolymer optical analysis apparatus according to Claim 1, wherein the external light is applied to the electrically conductive thin film to cause the electrically conductive thin film to produce a near field at an end portion facing an opening portion of the nanopore, whereby Raman scattered light from the biopolymer entering the nanopore is produced.

5. The biopolymer optical analysis apparatus according to Claim 1, wherein the electrically conductive thin film has an end portion having an acute angle, and the end portion having the acute angle is disposed so as to face the opening portion of the nanopore.

6. The biopolymer optical analysis apparatus according to Claim 1, wherein the characteristic analysis chip has at least two electrically conductive thin films for each of the nanopores, and the at least two electrically conductive thin films are opposed to each other with the opening portion of the nanopore interposed therebetween.

7. The biopolymer optical analysis apparatus according to Claim 1, wherein the electrically conductive thin film is formed of a metal.

8. The biopolymer optical analysis apparatus according to Claim 1, wherein the electrically conductive thin film is formed of graphite.

9. The biopolymer optical analysis apparatus according to Claim 1, wherein the thickness of the electrically conductive thin film is 0.1 nm to 10 nm.

10. The biopolymer optical analysis apparatus according to Claim 1, wherein the electrically conductive thin film is disposed at an intermediate depth in a direction along a center axis of the nanopore in the solid substrate.

11. The biopolymer optical analysis apparatus according to Claim 1, wherein a depth of each of the nanopores is equal to or larger than three times the monomer unit constituting the biopolymer.

12. The biopolymer optical analysis apparatus according to Claim 1, further comprising a data processing section that analyzes characteristics of the biopolymer from the ratios of the intensities of Raman scattered light detected in the detection device.

13. The biopolymer optical analysis apparatus according to Claim 1, further comprising a specimen moving mechanism that causes monomers in the biopolymer to enter the nanopore on a unit-by-unit basis.

14. The biopolymer optical analysis apparatus according to Claim 1, wherein the biopolymer is selected from the group consisting of a nucleic acid, a peptide nucleic acid, a protein, a sugar chain, and an aptamer.

15. The biopolymer optical analysis apparatus according to Claim 1, wherein analysis of characteristics of the biopolymer is determination of a base sequence of a nucleic acid.

16. A biopolymer optical analysis apparatus comprising:
a biopolymer characteristic analysis chip comprising a solid substrate and a plurality of pairs of electrically conductive thin films disposed on a surface of the solid substrate, wherein each pair of the electrically conductive thin films is disposed in a state of being opposed to each other with a certain distance;
a light source and an irradiation optical system for producing Raman scattered lights from a biopolymer; and
a detection device comprising a Raman scattered light collecting system, a separating component that splits collected light into transmitted light and reflected light at different ratios for each of prescribed wavelength bands, an image-forming optical system through which the split lights form images on a detector, and a two-dimensional detector for detecting the lights forming the images,
wherein external light is applied from the light source and the irradiation optical system to the characteristic analysis chip, and Raman scattered lights from the biopolymer in the analysis chip are detected in the detection device.

17. A biopolymer characteristic analysis method comprising the steps of:
in the biopolymer optical analysis apparatus according to Claim 1, applying external light to a characteristic analysis chip to produce Raman scattered lights from a biopolymer entering nanopores;
detecting the Raman scattered lights in a detection device; and
analyzing characteristics of the biopolymer on the basis of results of detection of the Raman scattered lights.

18. The biopolymer characteristic analysis method according to Claim 17, wherein the biopolymer is selected from the group consisting of a nucleic acid, a peptide nucleic acid, a protein, a sugar chain, and an aptamer.

19. The biopolymer characteristic analysis method according to Claim 17, wherein a base sequence of a nucleic acid is determined.

20. A biopolymer characteristic analysis chip comprising a solid substrate and at least one channel provided on the solid substrate, wherein by irradiation with external light, Raman scattering from a biopolymer entering the channel is produced.

21. The biopolymer characteristic analysis chip according to Claim 20, wherein the channel is formed by an electrically conductive thin film.

22. The biopolymer characteristic analysis chip according to Claim 21, wherein a projecting shape having a pointed end is formed in at least one place in the channel, and the projecting shape is formed on one of two wall surfaces of the channel on opposite sides of the flow direction of the channel.

23. The biopolymer characteristic analysis chip according to Claim 21, wherein projecting shapes each having a pointed end are formed in at least two places in the channel, and the projecting shapes are formed by being opposed from two wall surfaces of the channel on opposite sides of the flow direction of the channel.

24. The biopolymer characteristic analysis chip according to Claim 22 or 23, wherein the projecting shape produces a near field and the Raman scattering is produced by using the near field.

25. The biopolymer characteristic analysis chip according to Claim 21, wherein at least one row of metal nanoparticles is fixed across the channel at least in one place in the channel.

26. The biopolymer characteristic analysis chip according to Claim 25, wherein the metal nanoparticles produce a near field and the Raman scattering is produced by using the near field.

27. The biopolymer characteristic analysis chip according to Claim 21, wherein at least one row of metal nanostructures is fixed across the channel at least in one place in the channel.

28. The biopolymer characteristic analysis chip according to Claim 27, wherein the metal nanostructures produce a near field and the Raman scattering is produced by using the near field.
